# EUROPEAN PATENT SPECIFICATION

(11) **EP 2 866 810 B1**
(45) Date of publication and mention of the grant of the patent: **03.10.2018**
(21) Application number: 13810751.1
(22) Date of filing: 27.06.2013
(51) Int. Cl.: A61K 31/5685, A61K 31/56, A61P 35/00

(54) **INDUCTION OF ESTROGEN RECEPTOR BETA BY CHOLESTEROL BIOSYNTHESIS INHIBITORS AND METHODS OF TREATMENT OF CANCER**
INDUKTION DES ÖSTROGENREZEPTORS BETA DURCH CHOLESTEROL-BIOSYNTHESE-HEMMER SOWIE VERFAHREN ZUR BEHANDLUNG VON KREBS
INDUCTION DU RÉCEPTEUR BÊTA DES STROGÈNES PAR DES INHIBITEURS DE LA BIOSYNTHÈSE DU CHOLESTÉROL ET MÉTHODES DE TRAITEMENT DU CANCER

(30) Priority: 27.06.2012 US 201261665116 P
(43) Date of publication of application: 06.05.2015
(73) Proprietor: The Curators Of The University Of Missouri, Columbia, MO 65211-2015 (US)
(72) Inventor: HYDER, Salman, Columbia, MO 65211-2015 (US); LIANG, Yayun, Columbia, MO 65211-2015 (US)
(74) Representative: Greaves Brewster LLP
(86) International application number: PCT/US2013/048231
(87) International publication number: WO 2014/004854

(56) References cited:
- WO-A1-2013/059772
- WO-A2-2012/092114
- US-A1- 2010 092 479
- SAM Z GRINTER ET AL: "An inverse docking approach for identifying new potential anti-cancer targets", JOURNAL OF MOLECULAR GRAPHICS AND MODELLING, ELSEVIER SCIENCE, NEW YORK, NY, US, vol. 29, no. 6, 6 January 2011 (2011-01-06), pages 795-799, XP028157956, ISSN: 1093-3263, DOI: 10.1016/J.JMGM.2011.01.002 [retrieved on 2011-01-19]
- OWENS, C. M. ET AL.: 'Chemical combinations elucidate pathway interactions and regulation relevant to Hepatitis C replication' MOLECULAR SYSTEMS BIOLOGY vol. 6, no. 375, 2010, XP055047969
- LENHART, A. ET AL.: 'Crystal structure of a squalene cyclase in complex with the potential anticholesteremic drug Ro48-8071' CHEMISTRY & BIOLOGY vol. 9, 2002, pages 639 - 645, XP026921882
- MEJIA-POUS, C. ET AL.: 'Cholesterol synthesis-related enzyme oxidosqualene cyclase is required to maintain self-renewal in primary erythroid progenitors' CELL PROLIFERATION vol. 44, 2011, pages 441 - 452, XP055183597

## Description

This application claims the benefit of priority of United States provisional application No. 61/665,116, filed on June 27, 2012,

### GRANT STATEMENT

The invention was made with Government support under Grants No. R56CA86916 awarded by the National Institutes of Health. The US Government has certain rights in the invention.

Disclosed herein is a new method for the treatment of cancer, methods of using drugs in combination to improve cancer treatment, and combination therapies for the treatment of cancers including triple-negative breast cancer.

Estrogen receptors ("ER") have long been targeted in cancer therapy, particularly breast cancer therapy. There are two broad classes of receptors, each of which has further subtypes. Estrogen receptor-alpha (ERα) is a pro-proliferative protein; it induces cell proliferation and tumor growth. ERβ is a negative regulator of cell growth in breast cancer cells. Whereas ERα expression increases during breast tumorigenesis, particularly during the early stages, ERβ decreases. ERα and ERβ preferentially form functional heterodimers that bind DNA with an affinity similar to that of ERα homodimers, and greater than that of ERβ homodimers, and ERβ is thought to act as a negative regulator of ERα transcriptional activity. Thus, ERβ agonists have been explored as potential anti-cancer agents on the theory that promoting ERβ activity would lead to tumor suppression.

ERβ is widely expressed in the human body, having been detected in tumors of the colon, esophagus, stomach, brain, lung, prostate, testis, pancreas, and blood vessels, and the role of ERβ in other cancers is well-documented. For example, loss of ERβ expression is associated with advanced stages of not only breast but also colon and prostate cancers. ERβ protein expression also decreases progressively with more invasive melanomas. Selective ERβ agonists have also demonstrated antiproliferative and proapoptotic effects in Hodgkin's lymphoma cell lines and xenograft studies. Finally, ERβ agonists also promote both expression and tumor-suppressive functions of ERβ in glioma cells. Overall, these discoveries have prompted a great deal of research into ERβ modulators, agonists in particular.

Estrogen, of course, is a steroid hormone that regulates a broad array of bodily processes, so it is not surprising that estrogen receptors have been implicated in processes other than cancer. For example, recent data suggest that one function of ERβ may be to modulate the immune response, and that ERβ-selective ligands may be therapeutically useful agents to treat chronic intestinal and joint inflammation. Additionally, ERβ modulation had been suggested as a therapy for endometriosis and metabolic diseases such as obesity and metabolic syndrome. And although ERα and ERβ are expressed in the arterial wall and changes in expression are seen during the progression of atherosclerosis, the nature of each's participation warrants further investigation. For example, expression of ERβ in humans correlates with coronary calcification and atherosclerosis, yet expression of ERα and ERβ in the human aorta decreases with the progression of atherosclerosis.

Disclosed herein are methods and compositions related to the discovery that cholesterol biosynthesis inhibitors induce the anti-proliferative protein, estrogen receptor beta (ERβ), in both ERα-positive and ERα-negative breast cancer cell lines, including triple negative cells. Cholesterol biosynthesis inhibitors potently reduce the growth of breast cancer cells, and abolish pro-proliferative ERα in ERα-positive breast cancer cells. However, the combination of cholesterol biosynthesis inhibitors of OSC together with ERβ agonists additively arrested the growth of breast cancer cells than individual ligands used alone. This effect was particularly profound in the triple-negative cells. Additionally, down-regulation of ERβ using siRNA knock down experiments in tumor cells prevents the cell-killing effects mediated by cholesterol biosynthesis inhibitors.

Accordingly, disclosed herein is a method of treatment of cancer by sensitizing cells to chemotherapeutics by inducing ERβ. Although this method may be practiced in any tissue and cell type in which ERβ signaling is implicated, in one embodiment, the cancer is breast cancer. In a further embodiment, the cancer is ERβ-negative breast cancer, and in a yet further embodiment, the cancer is triple-negative breast cancer (i.e., negative for PR, ERα, and HER-2. In another embodiment, the cancer is ovarian cancer. In certain embodiments, the inducing is done by the administration of a cholesterol biosynthesis inhibitor. In further embodiments, the cholesterol biosynthesis inhibitor is an inhibitor of oxidosqualene cyclase ("OSC"), such as Ro 48-8071.

Also disclosed is a combination therapy for the treatment of cancer, comprising a cholesterol biosynthesis inhibitor and an ERβ agonist. In one embodiment, the cancer is breast cancer. In a further embodiment, the cancer is ERβ-negative breast cancer, and in a yet further embodiment, the cancer is triple-negative breast cancer (i.e., negative for PR, ERα, and HER-2. In another embodiment, the cancer is ovarian cancer. In further embodiments, the cholesterol biosynthesis inhibitor is an inhibitor of oxidosqualene cyclase ("OSC"), such as Ro 48-8071.

The cancer to be treated by the methods and compositions disclosed herein can be of different types and/or origins, for example breast, prostate, lung, colon, ovary, pancreatic, liver, thyroid, stomach, uterine, lymphoma, brain, skin, kidney, mouth, throat, tongue, nasal, esophageal, and bladder cancer, as well as leukemias and lymphomas, and the drug resistant phenotypes thereof.

### BRIEF DESCRIPTION OF THE DRAWINGS

FIG. 1. Effect of Ro 48-8071 treatment on expression of ERα, ERβ and Bcl-2 protein in T47-D breast cancer cells.
FIG. 2. Effect of Ro 48-8071 treatment on expression of ERα and ERβ protein in BT-474 breast cancer cells.
FIG. 3. Effect of low dose Ro 48-8071 on expression of ERα and ERβ.
FIG. 4. Ro 48-8071 stimulates expression of ERβ in ERα-negative cell lines (triple-negative).

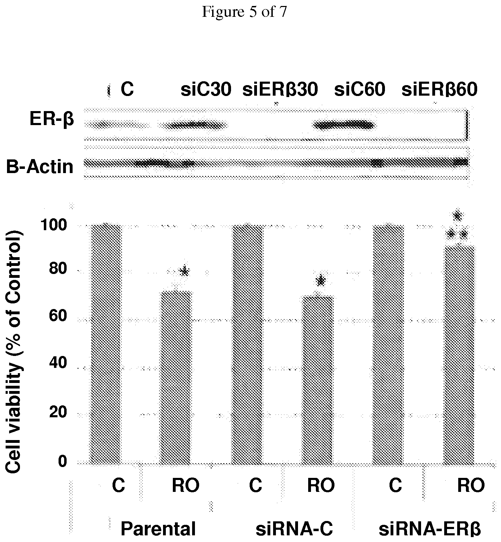

FIG. 5. ERβ knockdown with ERβ siRNA blocked the anti-proliferative effect of Ro 48-8071. T47-D cells were treated with either ERβ siRNA (30 and 60 nM; siERβ, Santa Cruz), scrambled RNA (siC) or transfection reagent alone (C) for 72h (upper panel). In the group treated with 60 nM siRNA, cells were treated with Ro 48-8071 (10 µM) or vehicle alone (C) for 48 h (lower panel) and tumor cell viability was determined using SRB assay as described. In the lower panel, * indicates significantly different from vehicle controls, and ** indicates significantly different from Ro 48-8071 treated samples in scrambled RNA group (siRNA-C) and parental cell group (p < 0.001; ANOVA). These results demonstrate that the anti-proliferative effects of Ro 48-8071 in breast cancer cells is reduced when the levels of ERβ are reduced in breast cancer cells.
FIG. 6. ERβ agonist potentiates the ability of Ro 48-8071 to reduce viability ofBT-474 cells. BT-474 cells were treated with 10 µM Ro 48-8071 ± 1 µM DPN, or with DPN alone. Cell viability was determined after 48 h using the SRB assay. In Fig. 6, * indicates significantly different from control, and ** indicates significantly different from Ro 48-8071 and DPN treated samples (p<0.001, ANOVA).
FIG. 7.. PHTPP ("PH"), an ERβ antagonist, blocks the anti-proliferative effect of Ro 48-8071. BT-474 cells treated with 10 µM Ro 48-8071 for 24 h ± 10 or 100 nM PH, or with PHTPP alone. Cell viability was determined after 24 h as described previously. In Fig. 7, * indicates significantly different from control, ** indicates significantly different from Ro 48-8071-treated sample, and *** indicates significantly different from control (p<0.001, ANOVA). The anti-proliferative effects of Ro 48-8071 in breast cancer cells is reduced when it is combined with ERβ antagonist.

### DETAILED DESCRIPTION

Accordingly, provided herein is a compostion for use in a method of treatment of cancer in a subject, comprising:
i) administering an amount of cholesterol biosynthesis inhibitor sufficient to induce ERβ in cancer cells; and
ii) administering a therapeutically effective amount of a selective or nonselective ERβ agonist;
wherein these steps may be performed sequentially or concurrently.

In certain embodiments of the method disclosed in paragraph [0018], the cholesterol biosynthesis inhibitor is an inhibitor of OSC. In other embodiments, the cholesterol biosynthesis inhibitor is an inhibitor of squalene monooxygenase. In other embodiments, the cholesterol biosynthesis inhibitor is an inhibitor of squalene synthase. In other embodiments, the cholesterol biosynthesis inhibitor is an inhibitor of farnesyl pyrophosphate synthase. In other embodiments, the cholesterol biosynthesis inhibitor is an inhibitor of geranyl pyrophosphate synthase, mevalonate-5-pyrophosphate decarboxylase, phosphomevalonate kinase, or mevalonate kinase.

In certain embodiments of the method disclosed in paragraph [0019], the inhibitor of OSC is a compound of Formula I, II, or III, disclosed in any of paragraphs [0083]-[0131] below. In certain embodiments, the inhibitor of OSC is chosen from the compounds disclosed in Table 1 below. In further embodiments, the inhibitor of OSC is chosen from the compounds disclosed in Table 1 below which have an IC₅₀ of less than 100 nM. In further embodiments, the inhibitor of OSC is chosen from the compounds disclosed in Table 1 below which have an IC₅₀ of less than 40 nM. In further embodiments, the inhibitor of OSC is chosen from the compounds disclosed in Table 1 below which have an IC₅₀ of less than 10 nM.

In certain embodiments of the method disclosed in paragraph [0020], the inhibitor of OSC is Ro 48-8071.

In certain embodiments of the method disclosed in any of paragraphs [0018]-[0021], the cancer is breast cancer.

In certain embodiments of the method disclosed in paragraph [0022], the cancer is ER-negative breast cancer.

In certain embodiments of the method disclosed in paragraph [0022], the cancer is ERβ-negative breast cancer.

In certain embodiments of the method disclosed in paragraph [0022], the cancer is triple-negative breast cancer.

In certain embodiments of the method disclosed in any of paragraphs [0018]-[0021], the cancer is ovarian cancer.

In certain embodiments of the method disclosed in any of paragraphs [0018]-[0021], the cancer is prostate cancer.

In certain embodiments of the method disclosed in any of paragraphs [0018]-[0021], the cancer is lung cancer.

In certain embodiments of the method disclosed in any of paragraphs [0018]-[0028], the ERβ agonist is a selective ERβ agonist.

In certain embodiments of the method disclosed in any of paragraphs [0018]-[0028], the ERβ agonist is chosen from 3β-adiol, DPN, apigenin, ERB-041, FERB-033, liquirtigenin, and WAY-00005.

Also provided is a method as recited in any of paragraphs [0018]-[0030], additionally comprising administering an antihormone.

In certain embodiments of the method disclosed in paragraph [0031], the antihormone is an antiestrogen.

In certain embodiments of the method disclosed in paragraph [0032], the antiestrogen is chosen from tamoxifen and fulvestrant.

Also provided herein is a pharmaceutical composition comprising a cholesterol inhibitor and an ERβ agonist.

In certain embodiments of the pharmaceutical composition disclosed in paragraph [0034], the cholesterol inhibitor is an inhibitor of cholesterol biosynthesis.

In certain embodiments of the pharmaceutical composition disclosed in paragraph [0035], the cholesterol biosynthesis inhibitor is an inhibitor of OSC. In other embodiments, the cholesterol biosynthesis inhibitor is an inhibitor of squalene monooxygenase. In other embodiments, the cholesterol biosynthesis inhibitor is an inhibitor of squalene synthase. In other embodiments, the cholesterol biosynthesis inhibitor is an inhibitor of farnesyl pyrophosphate synthase. In other embodiments, the cholesterol biosynthesis inhibitor is an inhibitor of geranyl pyrophosphate synthase, mevalonate-5-pyrophosphate decarboxylase, phosphomevalonate kinase, or mevalonate kinase.

In certain embodiments of the pharmaceutical composition disclosed in paragraph [0036], the inhibitor of OSC is a compound of Formula I, II, or III, disclosed in any of paragraphs [0083]-[0131] below. In certain embodiments, the inhibitor of OSC is chosen from the compounds disclosed in Table 1 below. In further embodiments, the inhibitor of OSC is chosen from the compounds disclosed in Table 1 below which have an IC₅₀ of less than 100 nM. In further embodiments, the inhibitor of OSC is chosen from the compounds disclosed in Table 1 below which have an IC₅₀ of less than 40 nM. In further embodiments, the inhibitor of OSC is chosen from the compounds disclosed in Table 1 below which have an IC₅₀ of less than 10 nM.

In certain embodiments of the pharmaceutical composition disclosed in paragraph [0037], the inhibitor of OSC is Ro 48-8071.

In certain embodiments of the pharmaceutical composition disclosed in any of paragraphs [0034]-[0038], the ERβ agonist is a selective ERβ agonist.

In certain embodiments of the pharmaceutical composition disclosed in any of paragraphs [0034]-[0039], the ERβ agonist is chosen from 3β-adiol, DPN, apigenin, ERB-041, FERB-033, liquirtigenin, and WAY-00005.

Also provided herein is a compostion for us in a method of treatment of cancer in a subject, comprising:
i) administering an amount of cholesterol biosynthesis inhibitor sufficient to induce ERβ in cancer cells; and
ii) administering a therapeutically effective amount of an antihormone;
wherein these steps may be performed sequentially or concurrently.

In certain embodiments of the method disclosed in paragraph [0041], the cholesterol biosynthesis inhibitor is an inhibitor of OSC. In other embodiments, the cholesterol biosynthesis inhibitor is an inhibitor of squalene monooxygenase. In other embodiments, the cholesterol biosynthesis inhibitor is an inhibitor of squalene synthase. In other embodiments, the cholesterol biosynthesis inhibitor is an inhibitor of farnesyl pyrophosphate synthase. In other embodiments, the cholesterol biosynthesis inhibitor is an inhibitor of geranyl pyrophosphate synthase, mevalonate-5-pyrophosphate decarboxylase, phosphomevalonate kinase, or mevalonate kinase.

In certain embodiments of the method disclosed in paragraph [0042], the inhibitor of OSC is a compound of Formula I, II, or III, disclosed in any of paragraphs [0083]-[0131] below. In certain embodiments, the inhibitor of OSC is chosen from the compounds disclosed in Table 1 below. In further embodiments, the inhibitor of OSC is chosen from the compounds disclosed in Table 1 below which have an IC₅₀ of less than 100 nM. In further embodiments, the inhibitor of OSC is chosen from the compounds disclosed in Table 1 below which have an IC₅₀ of less than 40 nM. In further embodiments, the inhibitor of OSC is chosen from the compounds disclosed in Table 1 below which have an IC₅₀ of less than 10 nM.

In certain embodiments of the method disclosed in paragraph [0043], the inhibitor of OSC is Ro 48-8071.

In certain embodiments of the method disclosed in any of paragraphs [0041]-[0044], the cancer is breast cancer.

In certain embodiments of the method disclosed in paragraph [0045], the cancer is ER-negative breast cancer.

In certain embodiments of the method disclosed in paragraph [0046], the cancer is ERβ-negative breast cancer.

In certain embodiments of the method disclosed in paragraph [0045], the cancer is triple-negative breast cancer.

In certain embodiments of the method disclosed in any of paragraphs [0041]-[0044], the cancer is ovarian cancer.

In certain embodiments of the method disclosed in any of paragraphs [0041]-[0044], the cancer is prostate cancer.

In certain embodiments of the method disclosed in any of paragraphs [0041]-[0044], wherein the cancer is lung cancer.

In certain embodiments of the method disclosed in any of paragraphs [0041]-[0051], the antihormone is an antiestrogen.

In certain embodiments of the method disclosed in paragraph [0052], the antiestrogen is chosen from tamoxifen and fulvestrant.

Also provided herein is a compostion for use in a method of treatment of a disease responsive to chemotherapy, comprising:
i) administering an amount of cholesterol biosynthesis inhibitor sufficient to induce ERβ in cancer cells; and
ii) administering a subtherapeutic amount of a chemotherapeutic drug;
wherein these steps may be performed sequentially or concurrently.

In certain embodiments of the method disclosed in paragraph [0054], the cholesterol biosynthesis inhibitor is an inhibitor of OSC. In other embodiments, the cholesterol biosynthesis inhibitor is an inhibitor of squalene monooxygenase. In other embodiments, the cholesterol biosynthesis inhibitor is an inhibitor of squalene synthase. In other embodiments, the cholesterol biosynthesis inhibitor is an inhibitor of farnesyl pyrophosphate synthase. In other embodiments, the cholesterol biosynthesis inhibitor is an inhibitor of geranyl pyrophosphate synthase, mevalonate-5-pyrophosphate decarboxylase, phosphomevalonate kinase, or mevalonate kinase.

In certain embodiments of the method disclosed in paragraph [0055], the inhibitor of OSC is a compound of Formula I, II, or III, disclosed in any of paragraphs [0083]-[0131] below. In certain embodiments, the inhibitor of OSC is chosen from the compounds disclosed in Table 1 below. In further embodiments, the inhibitor of OSC is chosen from the compounds disclosed in Table 1 below which have an IC₅₀ of less than 100 nM. In further embodiments, the inhibitor of OSC is chosen from the compounds disclosed in Table 1 below which have an IC₅₀ of less than 40 nM. In further embodiments, the inhibitor of OSC is chosen from the compounds disclosed in Table 1 below which have an IC₅₀ of less than 10 nM.

In certain embodiments of the method disclosed in paragraph [0056], the inhibitor of OSC is Ro 48-8071.

In certain embodiments of the method disclosed in any of paragraphs [0054]-[0057], the disease is cancer.

In certain embodiments of the method disclosed in paragraph [0058], the cancer is breast cancer.

In certain embodiments of the method disclosed in paragraph [0059], the cancer is ER-negative breast cancer.

In certain embodiments of the method disclosed in paragraph [0060], the cancer is ERβ-negative breast cancer.

In certain embodiments of the method disclosed in paragraph [0059], the cancer is triple-negative breast cancer.

In certain embodiments of the method disclosed in paragraph [0058], the cancer is ovarian cancer.

In certain embodiments of the method disclosed in paragraph [0058], the cancer is prostate cancer.

In certain embodiments of the method disclosed in paragraph [0058], wherein the cancer is lung cancer.

In certain embodiments of the method disclosed in any of paragraphs [0054]-[0065], the chemotherapeutic drug is chosen from doxorubicin and docetaxel.

Also provided herein is a pharmaceutical composition comprising a cholesterol inhibitor and an antihormone.

In certain embodiments of the pharmaceutical composition disclosed in paragraph [0067], the cholesterol inhibitor is an inhibitor of cholesterol biosynthesis.

In certain embodiments of the pharmaceutical composition disclosed in paragraph [0068], the cholesterol biosynthesis inhibitor is an inhibitor of OSC. In other embodiments, the cholesterol biosynthesis inhibitor is an inhibitor of squalene monooxygenase. In other embodiments, the cholesterol biosynthesis inhibitor is an inhibitor of squalene synthase. In other embodiments, the cholesterol biosynthesis inhibitor is an inhibitor of farnesyl pyrophosphate synthase. In other embodiments, the cholesterol biosynthesis inhibitor is an inhibitor of geranyl pyrophosphate synthase, mevalonate-5-pyrophosphate decarboxylase, phosphomevalonate kinase, or mevalonate kinase.

In certain embodiments of the pharmaceutical composition disclosed in paragraph [0069], the inhibitor of OSC is a compound of Formula I, II, or III, disclosed in any of paragraphs [0083]-[0131] below. In certain embodiments, the inhibitor of OSC is chosen from the compounds disclosed in Table 1 below. In further embodiments, the inhibitor of OSC is chosen from the compounds disclosed in Table 1 below which have an IC₅₀ of less than 100 nM. In further embodiments, the inhibitor of OSC is chosen from the compounds disclosed in Table 1 below which have an IC₅₀ of less than 40 nM. In further embodiments, the inhibitor of OSC is chosen from the compounds disclosed in Table 1 below which have an IC₅₀ of less than 10 nM.

In certain embodiments of the pharmaceutical composition disclosed in paragraph [0070], the inhibitor of OSC is Ro 48-8071.

In certain embodiments of the pharmaceutical composition disclosed in any of paragraphs [0067]-[007i], the antihormone is an antiestrogen.

In certain embodiments of the pharmaceutical composition disclosed in paragraph [0072] the antiestrogen is chosen from tamoxifen and fulvestrant.

Also provided herein is a kit comprising:
i) a cholesterol biosynthesis inhibitor formulated for administration to a patient;
ii) a therapeutically effective amount of a selective or nonselective ERβ agonist formulated for administration to a patient;
iii) instructions for administration to a patient.

In certain embodiments of the kit disclosed in paragraph [0074], the cholesterol biosynthesis inhibitor is an inhibitor of OSC. In other embodiments, the cholesterol biosynthesis inhibitor is an inhibitor of squalene monooxygenase. In other embodiments, the cholesterol biosynthesis inhibitor is an inhibitor of squalene synthase. In other embodiments, the cholesterol biosynthesis inhibitor is an inhibitor of farnesyl pyrophosphate synthase. In other embodiments, the cholesterol biosynthesis inhibitor is an inhibitor of geranyl pyrophosphate synthase, mevalonate-5-pyrophosphate decarboxylase, phosphomevalonate kinase, or mevalonate kinase.

In certain embodiments of the kit disclosed in paragraph [0075], the inhibitor of OSC is a compound of Formula I, II, or III, disclosed in any of paragraphs [0083]-[0131] below. In certain embodiments, the inhibitor of OSC is chosen from the compounds disclosed in Table 1 below. In further embodiments, the inhibitor of OSC is chosen from the compounds disclosed in Table 1 below which have an IC₅₀ of less than 100 nM. In further embodiments, the inhibitor of OSC is chosen from the compounds disclosed in Table 1 below which have an IC₅₀ of less than 40 nM. In further embodiments, the inhibitor of OSC is chosen from the compounds disclosed in Table 1 below which have an IC₅₀ of less than 10 nM.

In certain embodiments of the kit disclosed in paragraph [0076], the inhibitor of OSC is Ro 48-8071.

In certain embodiments of the kit disclosed in any of paragraphs [0074]-[0077], the ERβ agonist is a selective ERβ agonist.

In certain embodiments of the kit disclosed in any of paragraphs [0074]-[0078], the ERβ agonist is chosen from 3β-adiol, DPN, apigenin, ERB-041, FERB-033, liquirtigenin, and WAY-00005.

Also provided is a kit as recited in any of paragraphs [0074]-[0079], additionally comprising administering an antihormone.

In certain embodiments of the kit disclosed in paragraph [0080], the antihormone is an antiestrogen.

In certain embodiments of the kit disclosed in paragraph [0081], the antiestrogen is chosen from tamoxifen and fulvestrant.

The enzyme oxidosqualene cyclase ('OSC') is known to be a part of the cholesterol biosynthetic pathway, whereby OSC converts oxidosqualene to lanosterol forming the steroid scaffold.

Provided herein is a class of compounds including a tertiary amine linked with aromatic ring structures, as inhibitors of OSC and/or cholesterol biosynthesis for suppressing the viability of cancer cells is disclosed herein. In certain embodiments, inhibitors of OSC and/or cholesterol biosynthesis have the Formula I: 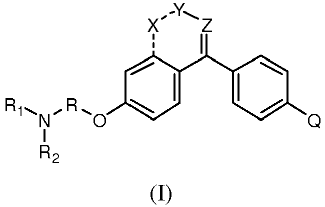 or a salt thereof, wherein:
X is chosen from hydrogen, halogen, O, NR₃R₄, S, CH₂, and CH;
Y is chosen from null, a bond, O and CH;
Z is chosen from O, N, and CH;
dashed bonds may be present or absent; if present, the bond may be single or double as valency allows;
R, R₁ and R₂ are independently chosen from alkyl, alkene, aryl, alkyne, cycloalkyl, and alkylcycloalkylalkyl, any of which may be optionally substituted;
R₃ and R₄ are independently chosen from a bond, hydrogen, lower alkyl, lower alkene, lower alkyne, aryl, and cycloalkyl, any of which may be optionally substituted; and
Q is chosen from bromine, chlorine and fluorine.

In certain embodiments are provided compounds of Formula I, wherein X is NH₂.

In certain embodiments are provided compounds of Formula I, wherein X is fluorine.

In certain embodiments are provided compounds of Formula I as disclosed in any of paragraphs [0084]-[0086], wherein R is alkyl.

In certain embodiments are provided compounds of Formula I as disclosed in any of paragraphs [0084]-[0087], wherein R₁ and R₂ are chosen from alkyl and alkene.

In certain embodiments are provided compounds of Formula I as disclosed in paragraph [0088], wherein R₁ and R₂ are chosen from lower alkyl and lower alkene.

In certain embodiments are provided compounds of Formula I as disclosed in paragraph [0089], wherein R₁ is lower alkyl.

In certain embodiments are provided compounds of Formula I as disclosed in paragraph [0090], wherein R₁ is methyl.

In certain embodiments are provided compounds of Formula I as disclosed in any of paragraphs [0084]-[0091], wherein R₂ is alkene.

In certain embodiments are provided compounds of Formula I as disclosed in paragraph [0092], wherein R₂ is lower alkene.

In certain embodiments are provided compounds of Formula I as disclosed in paragraph [0093], wherein R₂ is propene.

In certain embodiments are provided compounds of Formula I as disclosed in any of paragraphs [0084]-[0094], wherein R₂ is alkyl.

In certain embodiments are provided compounds of Formula I as disclosed in paragraph [0095], wherein R₂ is lower alkyl.

In certain embodiments are provided compounds of Formula I as disclosed in paragraph [0096], wherein R₂ is lower cycloalkyl.

In certain embodiments are provided compounds of Formula I as disclosed in paragraph [0097], wherein R₂ is cyclopropyl.

In further embodiments, new inhibitors have the Formula II: 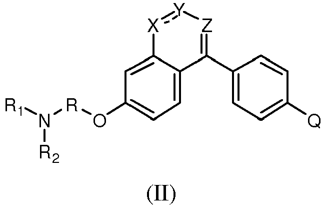 or a salt thereof, wherein:
X is chosen from O, N, NR₃, S, CH, AND CH₂;
Y is chosen from null, a bond, O and CH;
Z is chosen from O, N, and CH; and
the dashed bond may be present or absent;
R, R₁, and R₂ are independently chosen from alkyl, alkene, aryl, alkyne, cycloalkyl, and alkylcycloalkylalkyl, any of which may be optionally substituted;
R₃ is chosen from a bond, hydrogen, lower alkyl, lower alkene, lower alkyne, aryl, and cycloalkyl, any of which may be optionally substituted; and
Q is chosen from bromine, chlorine and fluorine.

In certain embodiments are provided compounds of Formula II, wherein R is alkyl.

In certain embodiments are provided compounds of Formula II as disclosed in any of paragraphs [0099]-[00100], wherein R is lower alkyl.

In certain embodiments are provided compounds of Formula II as disclosed in any of paragraphs [0099]-[00101], wherein R₁ and R₂ are chosen from alkyl and alkene.

In certain embodiments are provided compounds of Formula II as disclosed in paragraph [00102], wherein R₁ and R₂ are chosen from lower alkyl and lower alkene.

In certain embodiments are provided compounds of Formula II as disclosed in paragraph [00103], wherein R₁ is lower alkyl.

In certain embodiments are provided compounds of Formula II as disclosed in, paragraph [00104] wherein R₁ is methyl.

In certain embodiments are provided compounds of Formula II as disclosed in any of paragraphs [0099]-[00105] wherein R₂ is alkene.

In certain embodiments are provided compounds of Formula II as disclosed in paragraph [00107], wherein R₂ is lower alkene.

In certain embodiments are provided compounds of Formula II as disclosed in paragraph [00108], wherein R₂ is propene.

In certain embodiments are provided compounds of Formula II as disclosed in any of paragraphs [0099]-[00105], wherein R₂ is alkyl.

In certain embodiments are provided compounds of Formula II as disclosed in paragraph [00109], wherein R₂ is lower alkyl.

In certain embodiments are provided compounds of Formula II as disclosed in paragraph [00110], wherein R₂ is lower cycloalkyl.

In certain embodiments are provided compounds of Formula II as disclosed in paragraph [00111], wherein R₂ is cyclopropyl.

In further embodiments, new inhibitors have the Formula III: 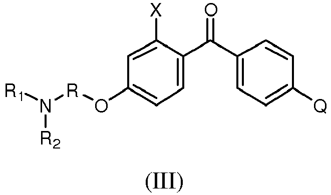 or a salt thereof, wherein:
X is chosen from hydrogen, halogen, and NR₃R₄;
R, R₁, and R₂ are independently chosen from alkyl, alkene, aryl, alkyne, cycloalkyl, and alkylcycloalkylalkyl, any of which may be optionally substituted;
R₃ and R₄ are independently chosen from a bond, hydrogen, lower alkyl, lower alkene, lower alkyne, aryl, and cycloalkyl, any of which may be optionally substituted; and
Q is chosen from bromine, chlorine and fluorine.

In certain embodiments are provided compounds of Formula III, wherein X is NH₂.

In certain embodiments are provided compounds of Formula III, wherein X is fluorine.

In certain embodiments are provided compounds of Formula III as disclosed in any of paragraphs [00113]-[00115], wherein R is alkyl.

In certain embodiments are provided compounds of Formula III as disclosed in any of paragraphs [00113]-[00116], wherein R₁ and R₂ are chosen from alkyl and alkene.

In certain embodiments are provided compounds of Formula III as disclosed in paragraph [00117], wherein R₁ and R₂ are chosen from lower alkyl and lower alkene.

In certain embodiments are provided compounds of Formula III as disclosed in paragraph [00118], wherein R₁ is lower alkyl.

In certain embodiments are provided compounds of Formula III as disclosed in paragraph [00119], wherein R₁ is methyl.

In certain embodiments are provided compounds of Formula III as disclosed in any of paragraphs [00113]-[00120], wherein R₂ is alkene.

In certain embodiments are provided compounds of Formula III as disclosed in paragraph [00121], wherein R₂ is lower alkene.

In certain embodiments are provided compounds of Formula III as disclosed in paragraph [00122], wherein R₂ is propene.

In certain embodiments are provided compounds of Formula III as disclosed in any of paragraphs [00113]-[00120], wherein R₂ is alkyl.

In certain embodiments are provided compounds of Formula III as disclosed in paragraph [00124], wherein R₂ is lower alkyl.

In certain embodiments are provided compounds of Formula III as disclosed in paragraph [00125], wherein R₂ is lower cycloalkyl.

In certain embodiments are provided compounds of Formula III as disclosed in paragraph [00126], wherein R₂ is cyclopropyl.

Examples of potent inhibitor of OSC of Formula I having containing a tertiary amine, a hexyloxy spacer and an unrestrained halophenyl group include Ro 48-8071, also known as (4-bromophenyl)[2-fluoro-4-[[6-(methyl-2-propenylamino)hexyl]oxy]phenyl]-methanone, or (4' - [6-(Allylmethylamino)hexyloxy] -4-bromo-2' -fluorobenzophenone fumarate).

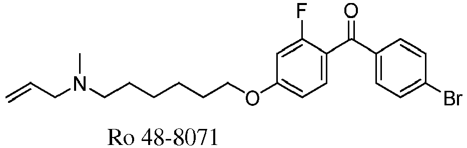

Ro 48-8071 is commercially available; see, e.g., Sigma-Aldrich, Product No. R2278. Although OSC, along with its inhibitors, has been studied as a target to reduce plasma cholesterol levels. [5] OSC has not previously been identified as a potential antitumor target.

Examples of analogues having an aromatic linker between the bromophenyl ring and the methoxyphenyl ring, or having a ketonic linker between the rings, are further given below:

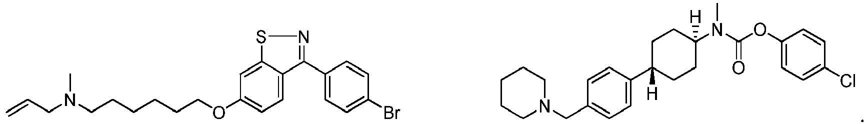

Additional examples of OSC inhibitors are given below.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety.

When ranges of values are disclosed, and the notation "from n₁ ... to n₂" is used, where n₁ and n₂ are the numbers, then unless otherwise specified, this notation is intended to include the numbers themselves and the range between them. This range may be integral or continuous between and including the end values. By way of example, the range "from 2 to 6 carbons" is intended to include two, three, four, five, and six carbons, since carbons come in integer units. Compare, by way of example, the range "from 1 to 3 µM (micromolar)," which is intended to include 1 µM, 3 µM, and everything in between to any number of significant figures (e.g., 1.255 µM, 2.1 µM, 2.9999 µM, etc.).

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

The term "alkenyl," as used herein, alone or in combination, refers to a straight-chain or branched-chain hydrocarbon group having one or more double bonds and containing from 2 to 20 carbon atoms. In certain embodiments, said alkenyl will comprise from 2 to 6 carbon atoms. The term "alkenylene" refers to a carbon-carbon double bond system attached at two or more positions such as ethenylene [(-CH=CH-),(-C::C-)]. Examples of suitable alkenyl groups include ethenyl, propenyl, 2-methylpropenyl, 1,4-butadienyl and the like. Unless otherwise specified, the term "alkenyl" may include "alkenylene" groups.

The term "alkyl," as used herein, alone or in combination, refers to a straight-chain or branched-chain alkyl group containing from 1 to 20 carbon atoms. In certain embodiments, said alkyl will comprise from 1 to 10 carbon atoms. In further embodiments, said alkyl will comprise from 1 to 6 carbon atoms. Alkyl groups may be optionally substituted as defined herein. Examples of alkyl groups include methyl, ethyl, n-propyl, isopropyl, n-butyl, isobutyl, sec-butyl, tert-butyl, pentyl, iso-amyl, hexyl, octyl, noyl and the like. The term "alkylene," as used herein, alone or in combination, refers to a saturated aliphatic group derived from a straight or branched chain saturated hydrocarbon attached at two or more positions, such as methylene (-CH₂-). Unless otherwise specified, the term "alkyl" may include "alkylene" groups.

The term "alkynyl," as used herein, alone or in combination, refers to a straight-chain or branched chain hydrocarbon group having one or more triple bonds and containing from 2 to 20 carbon atoms. In certain embodiments, said alkynyl comprises from 2 to 6 carbon atoms. In further embodiments, said alkynyl comprises from 2 to 4 carbon atoms. The term "alkynylene" refers to a carbon-carbon triple bond attached at two positions such as ethynylene (-C:::C-, -C=C-). Examples of alkynyl groups include ethynyl, propynyl, hydroxypropynyl, butyn-1-yl, butyn-2-yl, pentyn-1-yl, 3-methylbutyn-1-yl, hexyn-2-yl, and the like. Unless otherwise specified, the term "alkynyl" may include "alkynylene" groups.

The term "aryl," as used herein, alone or in combination, means a carbocyclic aromatic system containing one, two or three rings wherein such polycyclic ring systems are fused together. The term "aryl" embraces aromatic groups such as phenyl, naphthyl, anthracenyl, and phenanthryl.

The term "bond" refers to a covalent linkage between two atoms, or two moieties when the atoms joined by the bond are considered to be part of larger substructure. A bond may be single, double, or triple unless otherwise specified. A dashed line between two atoms in a drawing of a molecule indicates that an additional bond may be present or absent at that position. When a ring or chain element is designated to be a bond, what is meant is that the flanking moieties have a covalent bond between them. For example, in the chain X-Y-Z, when Y is a bond, the chain collapses to X-Z.

The term "cycloalkyl," or, alternatively, "carbocycle," as used herein, alone or in combination, refers to a saturated or partially saturated monocyclic, bicyclic or tricyclic alkyl group wherein each cyclic moiety contains from 3 to 12 carbon atom ring members and which may optionally be a benzo fused ring system which is optionally substituted as defined herein. In certain embodiments, said cycloalkyl will comprise from 5 to 7 carbon atoms. Examples of such cycloalkyl groups include cyclopropyl, cyclobutyl, cyclopentyl, cyclohexyl, cycloheptyl, tetrahydronapthyl, indanyl, octahydronaphthyl, 2,3-dihydro-1H-indenyl, adamantyl and the like. "Bicyclic" and "tricyclic" as used herein are intended to include both fused ring systems, such as decahydronaphthalene, octahydronaphthalene as well as the multicyclic (multicentered) saturated or partially unsaturated type. The latter type of isomer is exemplified in general by, bicyclo[1,1,1]pentane, camphor, adamantane, and bicyclo[3,2,1]octane.

The term "halo," or "halogen," as used herein, alone or in combination, refers to fluorine, chlorine, bromine, or iodine.

The term "lower," as used herein, alone or in a combination, where not otherwise specifically defined, means containing from 1 to and including 6 carbon atoms.

Any definition herein may be used in combination with any other definition to describe a composite structural group. By convention, the trailing element of any such definition is that which attaches to the parent moiety. For example, the composite group alkylamido would represent an alkyl group attached to the parent molecule through an amido group, and the term alkoxyalkyl would represent an alkoxy group attached to the parent molecule through an alkyl group.

When a group is defined to be "null," what is meant is that said group is absent.

The term "optionally substituted" means the anteceding group may be substituted or unsubstituted. When substituted, the substituents of an "optionally substituted" group may include, without limitation, one or more substituents independently selected from the following groups or a particular designated set of groups, alone or in combination: lower alkyl, lower alkenyl, lower alkynyl, lower alkanoyl, lower heteroalkyl, lower heterocycloalkyl, lower haloalkyl, lower haloalkenyl, lower haloalkynyl, lower perhaloalkyl, lower perhaloalkoxy, lower cycloalkyl, phenyl, aryl, aryloxy, lower alkoxy, lower haloalkoxy, oxo, lower acyloxy, carbonyl, carboxyl, lower alkylcarbonyl, lower carboxyester, lower carboxamido, cyano, hydrogen, halogen, hydroxy, ester, acyl, amino, lower alkylamino, arylamino, amido, nitro, thiol, lower alkylthio, lower haloalkylthio, lower perhaloalkylthio, arylthio, sulfonate, sulfonic acid, trisubstituted silyl, N₃, SH, SCH₃, C(O)CH₃, CO₂CH₃, CO₂H, pyridinyl, thiophene, furanyl, lower carbamate, and lower urea. Two substituents may be joined together to form a fused five-, six-, or seven-membered carbocyclic or heterocyclic ring consisting of zero to three heteroatoms, for example forming methylenedioxy or ethylenedioxy. An optionally substituted group may be unsubstituted (e.g., -CH₂CH₃), fully substituted (e.g., -CF₂CF₃), monosubstituted (e.g., -CH₂CH₂F) or substituted at a level anywhere in-between fully substituted and monosubstituted (e.g., - CH₂CF₃). Where substituents are recited without qualification as to substitution, both substituted and unsubstituted forms are encompassed. Where a substituent is qualified as "substituted," the substituted form is specifically intended. Additionally, different sets of optional substituents to a particular moiety may be defined as needed; in these cases, the optional substitution will be as defined, often immediately following the phrase, "optionally substituted with."

As used herein, the term "modulate" means to increase or decrease the activity of a target or the amount of a substance.

As used herein, the term "increase" or the related terms "increased", "enhance" or "enhanced" refers to a statistically significant increase. For the avoidance of doubt, the terms generally refer to at least a 10% increase in a given parameter, and can encompass at least a 20% increase, 30% increase, 40% increase, 50% increase, 60% increase, 70% increase, 80% increase, 90% increase, 95% increase, 97% increase, 99% or even a 100% increase over the control value.

A "sample" as used herein refers to a biological material which can be tested, e.g., for the presence of OSC activity, or to determine if a test agent is capable of modulating the activity of OSC either in vitro, or inside a cell. Such samples may contain purified or semipurified, or non-purified preparations of OSC, for in vitro measurements. Samples may also comprise cells comprising intracellular OSC, for intracellular measurements of OSC activity. Samples of cells will typically contain buffers and salts to maintain physiological ionic strength and pH and be maintained at an appropriate temperature to preserve viability. Cells may be obtained from any source, including tissue culture, or tissue samples. In one aspect, such cells are mammalian cells. A sample may also include suitable control reagents (control samples).

The term "disease" as used herein is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The term "combination therapy" means the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the conditions or disorders described herein.

As used herein, an "amount sufficient to induce ERβ" means at least an amount sufficient to induce ERβ to a level detectable by assays known in the art. Such assays include well-known immunohistochemical assays for ERs such as those disclosed in Harvey JM et al., "Estrogen receptor status by immunohistochemistry is superior to the ligand-binding assay for predicting response to adjuvant endocrine therapy in breast cancer," J Clin Oncol. 1999 May;17(5):1474-81, and quantitative variations thereon. In further embodiments, the "amount sufficient to induce ERβ" is an amount at least statistically significant in comparison to, comparable untreated cells; such cells may be a patient's own or an acceptable reference standard. In yet further embodiments, the "amount sufficient to induce ERβ" is an amount that induces ERβ sufficient to permit treatment with an ERβ agonist (preferably, a therapeutically effective amount).

The phrase "therapeutically effective" is intended to qualify the amount of active ingredients used in the treatment of a disease or disorder. This amount will achieve the goal of reducing or eliminating the disease or disorder. In appropriate circumstances, a therapeutically effective amount may be approximated in cellular or in vivo assays. For example, a therapeutically effective amount of an ERβ agonist may mean, at minimum, an amount sufficient to detectably reduce cancer cell number or viability; preferably, the amount would reduce cancer cell number or viability to a statistically significant degree. Measurements of cancer cell number or viability may be taken both of lab-cultured cells and of cells taken from patients. Alternatively, for example, a therapeutically effective amount of an ERβ agonist may mean, at minimum, an amount sufficient to detectably (preferably, statistically significantly) shrink a tumor or reduce a relevant cancer biomarker. Alternatively, other measures of therapeutic efficacy may be used, such as delayed or reduced markers of disease progression, or mean survival.

The term "subtherapeutic amount" of a chemotherapeutic drug means an amount that would be below an accepted therapeutically effective amount. A subtherapeutic amount can be defined as an amount less than the FDA-approved dosage or dosages for a particular disease. Alternatively, taking into account that many drugs are used off-label, a subtherapeutic amount can be defined as an amount less than that typically prescribed by physicians for a particular disease. A sub-therapeutic amount may also take into account such factors as body mass, sex, age, renal or hepatic impairment, and other parameters which may affect the efficaciousness of a given amount of a particular drug. In certain embodiments, a subtherapeutic amount may be 70% of a therapeutically effective amount. In further embodiments, a subtherapeutic amount may be 60% of a therapeutically effective amount. In further embodiments, a subtherapeutic amount may be 50% of a therapeutically effective amount. In further embodiments, a subtherapeutic amount may be 40% of a therapeutically effective amount. In further embodiments, a subtherapeutic amount may be 30% of a therapeutically effective amount. In further embodiments, it may be even less. The compositions and methods disclosed herein may make a subtherapeutic dose of a chemotherapeutic agent a therapeutic one by sensitizing cells to chemotherapy, particularly via ERβ induction.

The term "therapeutically acceptable" refers to those compounds (or salts, polymorphs, prodrugs, tautomers, zwitterionic forms, etc.) which are suitable for use in contact with the tissues of patients without undue toxicity, irritation, and allergic response, are commensurate with a reasonable benefit/risk ratio, and are effective for their intended use.

As used herein, reference to "treatment" of a patient is intended to include prophylaxis. The term "patient" means all mammals including humans. Examples of patients include humans, cows, dogs, cats, goats, sheep, pigs, and rabbits. Preferably, the patient is a human.

The term "prodrug" refers to a compound that is made more active in vivo. Certain compounds disclosed herein may also exist as prodrugs, as described in Hydrolysis in Drug and Prodrug Metabolism : Chemistry, Biochemistry, and Enzymology (Testa, Bernard and Mayer, Joachim M. Wiley-VHCA, Zurich, Switzerland 2003). Prodrugs of the compounds described herein are structurally modified forms of the compound that readily undergo chemical changes under physiological conditions to provide the compound. Additionally, prodrugs can be converted to the compound by chemical or biochemical methods in an ex vivo environment. For example, prodrugs can be slowly converted to a compound when placed in a transdermal patch reservoir with a suitable enzyme or chemical reagent. Prodrugs are often useful because, in some situations, they may be easier to administer than the compound, or parent drug. They may, for instance, be bioavailable by oral administration whereas the parent drug is not. The prodrug may also have improved solubility in pharmaceutical compositions over the parent drug. A wide variety of prodrug derivatives are known in the art, such as those that rely on hydrolytic cleavage or oxidative activation of the prodrug. An example, without limitation, of a prodrug would be a compound which is administered as an ester (the "prodrug"), but then is metabolically hydrolyzed to the carboxylic acid, the active entity. Additional examples include peptidyl derivatives of a compound.

The compounds disclosed herein can exist as therapeutically acceptable salts. Suitable acid addition salts include those formed with both organic and inorganic acids, and will normally be pharmaceutically acceptable. However, salts of non-pharmaceutically acceptable salts may be of utility in the preparation and purification of the compound in question. Basic addition salts may also be formed and be pharmaceutically acceptable. Representative acid addition salts include acetate, adipate, alginate, L-ascorbate, aspartate, benzoate, benzenesulfonate (besylate), bisulfate, butyrate, camphorate, camphorsulfonate, citrate, digluconate, formate, fumarate, gentisate, glutarate, glycerophosphate, glycolate, hemisulfate, heptanoate, hexanoate, hippurate, hydrochloride, hydrobromide, hydroiodide, 2-hydroxyethansulfonate (isethionate), lactate, maleate, malonate, DL-mandelate, mesitylenesulfonate, methanesulfonate, naphthylenesulfonate, nicotinate, 2-naphthalenesulfonate, oxalate, pamoate, pectinate, persulfate, 3-phenylproprionate, phosphonate, picrate, pivalate, propionate, pyroglutamate, succinate, sulfonate, tartrate, L-tartrate, trichloroacetate, trifluoroacetate, phosphate, glutamate, bicarbonate, paratoluenesulfonate (p-tosylate), and undecanoate. Also, basic groups in the compounds disclosed herein can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. For a more complete discussion of the preparation and selection of salts, refer to Pharmaceutical Salts: Properties, Selection, and Use (Stahl, P. Heinrich. Wiley-VCHA, Zurich, Switzerland, 2002).

The term "therapeutically acceptable salt," as used herein, represents salts or zwitterionic forms of the compounds disclosed herein which are water or oil-soluble or dispersible and therapeutically acceptable as defined herein. The salts can be prepared during the final isolation and purification of the compounds or separately by reacting the appropriate compound in the form of the free base with a suitable acid. Examples of acids which can be employed to form therapeutically acceptable addition salts include inorganic acids such as hydrochloric, hydrobromic, sulfuric, and phosphoric, and organic acids such as oxalic, maleic, succinic, and citric. Salts can also be formed by coordination of the compounds with an alkali metal or alkaline earth ion.

Basic addition salts can be prepared during the final isolation and purification of the compounds, often by reacting a carboxy group with a suitable base such as the hydroxide, carbonate, or bicarbonate of a metal cation or with ammonia or an organic primary, secondary, or tertiary amine. The cations of therapeutically acceptable salts include lithium, sodium (e.g., NaOH), potassium (e.g., KOH), calcium (including Ca(OH)₂), magnesium (including Mg(OH)₂ and magnesium acetate), zinc, (including Zn(OH)₂ and zinc acetate) and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, *N,N*-dimethylaniline, N-methylpiperidine, *N*-methylmorpholine, dicyclohexylamine, procaine, dibenzylamine, *N,N-*dibenzylphenethylamine, 1-ephenamine, and *N,N*'-dibenzylethylenediamine. Other representative organic amines useful for the formation of base addition salts include ethylenediamine, ethanolamine, diethanolamine, piperidine, piperazine, choline hydroxide, hydroxyethyl morpholine, hydroxyethyl pyrrolidone, imidazole, n-methyl-d-glucamine, N, N'-dibenzylethylenediamine, N, N'-diethylethanolamine, N, N'-dimethylethanolamine, triethanolamine, and tromethamine. Basic amino acids such as l-glycine and l-arginine, and amino acids which may be zwitterionic at neutral pH, such as betaine (*N,N,N-*trimethylglycine) are also contemplated.

Salts disclosed herein may combine in 1:1 molar ratios, and in fact this is often how they are initially synthesized. However, it will be recognized by one of skill in the art that the stoichiometry of one ion in a salt to the other may be otherwise. Salts shown herein may be, for the sake of convenience in notation, shown in a 1:1 ratio; all possible stoichiometric arrangements are encompassed by the scope of the present invention.

The terms, "polymorphs" and "polymorphic forms" and related terms herein refer to crystal forms of the same molecule, and different polymorphs may have different physical properties such as, for example, melting temperatures, heats of fusion, solubilities, dissolution rates and/or vibrational spectra as a result of the arrangement or conformation of the molecules in the crystal lattice. The differences in physical properties exhibited by polymorphs affect pharmaceutical parameters such as storage stability, compressibility and density (important in formulation and product manufacturing), and dissolution rates (an important factor in bioavailability). Differences in stability can result from changes in chemical reactivity (e.g. differential oxidation, such that a dosage form discolors more rapidly when comprised of one polymorph than when comprised of another polymorph) or mechanical changes (e.g. tablets crumble on storage as a kinetically favored polymorph converts to thermodynamically more stable polymorph) or both (e. g., tablets of one polymorph are more susceptible to breakdown at high humidity). As a result of solubility/dissolution differences, in the extreme case, some polymorphic transitions may result in lack of potency or, at the other extreme, toxicity. In addition, the physical properties of the crystal may be important in processing, for example, one polymorph might be more likely to form solvates or might be difficult to filter and wash free of impurities (i.e., particle shape and size distribution might be different between polymorphs).

Polymorphs of a molecule can be obtained by a number of methods, as known in the art. Such methods include, but are not limited to, melt recrystallization, melt cooling, solvent recrystallization, desolvation, rapid evaporation, rapid cooling, slow cooling, vapor diffusion and sublimation.

While it may be possible for the compounds and prodrugs disclosed herein to be administered as the raw chemical, it is also possible to present them as a pharmaceutical formulation. Accordingly, provided herein are pharmaceutical formulations which comprise one or more of certain compounds and prodrugs disclosed herein, or one or more pharmaceutically acceptable salts, esters, amides, or solvates thereof, together with one or more pharmaceutically acceptable carriers thereof and optionally one or more other therapeutic ingredients. The carrier(s) must be "acceptable" in the sense of being compatible with the other ingredients of the formulation and not deleterious to the recipient thereof. Proper formulation is dependent upon the route of administration chosen. Any of the well-known techniques, carriers, and excipients may be used as suitable and as understood in the art; *e*.*g*., in Remington's Pharmaceutical Sciences. The pharmaceutical compositions disclosed herein may be manufactured in any manner known in the art, *e*.*g*., by means of conventional mixing, dissolving, granulating, dragee-making, levigating, emulsifying, encapsulating, entrapping or compression processes.

The formulations include those suitable for oral, parenteral (including subcutaneous, intradermal, intramuscular, intravenous, intraarticular, and intramedullary), intraperitoneal, transmucosal, transdermal, intranasal, rectal and topical (including dermal, buccal, sublingual and intraocular) administration although the most suitable route may depend upon for example the condition and disorder of the recipient. The formulations may conveniently be presented in unit dosage form and may be prepared by any of the methods well known in the art of pharmacy. Typically, these methods include the step of bringing into association a compound of the subject invention or a pharmaceutically acceptable salt, ester, amide, prodrug or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the compounds and prodrugs disclosed herein suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds and prodrugs may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

The compounds and prodrugs may be formulated for parenteral administration by injection, *e*.*g*., by bolus injection or continuous infusion. Formulations for injection may be presented in unit dosage form, *e*.*g*., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for parenteral administration include aqueous and non-aqueous (oily) sterile injection solutions of the active compounds and prodrugs which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds and prodrugs to allow for the preparation of highly concentrated solutions.

In addition to the formulations described previously, a compound or prodrug as disclosed herein may also be formulated as a depot preparation. Such long acting formulations may be administered by implantation (for example subcutaneously or intramuscularly) or by intramuscular injection. Thus, for example, the compounds and prodrugs may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in conventional manner. Such compositions may comprise the active ingredient in a flavored basis such as sucrose and acacia or tragacanth.

The compounds and prodrugs may also be formulated in rectal compositions such as suppositories or retention enemas, *e*.*g*., containing conventional suppository bases such as cocoa butter, polyethylene glycol, or other glycerides.

Certain compounds and prodrugs disclosed herein may be administered topically, that is by non-systemic administration. This includes the application of a compound disclosed herein externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semi-liquid preparations suitable for penetration through the skin to the site of inflammation such as gels, liniments, lotions, creams, ointments or pastes, and drops suitable for administration to the eye, ear or nose. The active ingredient for topical administration may comprise, for example, from 0.001% to 10% w/w (by weight) of the formulation. In certain embodiments, the active ingredient may comprise as much as 10% w/w. In other embodiments, it may comprise less than 5% w/w. In certain embodiments, the active ingredient may comprise from 2% w/w to 5% w/w. In other embodiments, it may comprise from 0.1% to 1% w/w of the formulation.

For administration by inhalation, compounds and prodrugs may be conveniently delivered from an insufflator, nebulizer pressurized packs or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the compounds and prodrugs disclosed herein may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflator.

Intranasal delivery, in particular, may be useful for delivering compounds to the CNS. It had been shown that intranasal drug administration is a noninvasive method of bypassing the blood-brain barrier (BBB) to deliver neurotrophins and other therapeutic agents to the brain and spinal cord. Delivery from the nose to the CNS occurs within minutes along both the olfactory and trigeminal neural pathways. Intranasal delivery occurs by an extracellular route and does not require that drugs bind to any receptor or undergo axonal transport. Intranasal delivery also targets the nasal associated lymphatic tissues (NALT) and deep cervical lymph nodes. In addition, intranasally administered therapeutics are observed at high levels in the blood vessel walls and perivascular spaces of the cerebrovasculature. Using this intranasal method in animal models, researchers have successfully reduced stroke damage, reversed Alzheimer's neurodegeneration, reduced anxiety, improved memory, stimulated cerebral neurogenesis, and treated brain tumors. In humans, intranasal insulin has been shown to improve memory in normal adults and patients with Alzheimer's disease. Hanson LR and Frey WH, 2nd, J Neuroimmune Pharmacol. 2007 Mar;2(1):81-6. Epub 2006 Sep 15.

Preferred unit dosage formulations are those containing an effective dose, as herein below recited, or an appropriate fraction thereof, of the active ingredient.

It should be understood that in addition to the ingredients particularly mentioned above, the formulations described above may include other agents conventional in the art having regard to the type of formulation in question, for example those suitable for oral administration may include flavoring agents.

Compounds and prodrugs may be administered orally or via injection at a dose of from 0.1 to 500 mg/kg per day. The dose range for adult humans is generally from 5 mg to 2 g/day. Tablets or other forms of presentation provided in discrete units may conveniently contain an amount of one or more compound or prodrug which is effective at such dosage or as a multiple of the same, for instance, units containing 5 mg to 500 mg, usually around 10 mg to 200 mg.

The amount of active ingredient that may be combined with the carrier materials to produce a single dosage form will vary depending upon the host treated and the particular mode of administration.

The compounds and prodrugs can be administered in various modes, e.g. orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the indication or condition being treated. Also, the route of administration may vary depending on the condition and its severity.

In certain instances, it may be appropriate to administer at least one of the compounds and prodrugs described herein (or a pharmaceutically acceptable salt or ester thereof) in combination with another therapeutic agent. By way of example only, if one of the side effects experienced by a patient upon receiving one of the compounds herein for the treatment of actinide poisoning is depletion of essential trace minerals required by the body for proper functioning, then it may be appropriate to administer a strong chelating agent in combination with supplements of essential trace minerals required by the body for proper functioning, for example zinc and magnesium, to replace those which will inadvertently be lost to chelation therapy. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced). Or, by way of example only, the benefit of experienced by a patient may be increased by administering one of the compounds described herein with another therapeutic agent (which also includes a therapeutic regimen) that also has therapeutic benefit. By way of example only, in a treatment for thalassemia involving administration of one of the compounds described herein, increased therapeutic benefit may result by also providing the patient with another therapeutic agent for thalassemia, for example deferoxamine. In any case, regardless of the disease, disorder or condition being treated, the overall benefit experienced by the patient may simply be additive of the two therapeutic agents or the patient may experience a synergistic benefit.

Specific, non-limiting examples of possible combination therapies include use of certain compounds disclosed herein with one or more agents chosen from: aromatase inhibitors, antiestrogens, anti-progestins, anti-androgens, or gonadorelin agonists, topoisomerase 1 and 2 inhibitors, microtubule active agents, alkylating agents, antineoplastic, antimetabolite, dacarbazine (DTIC), or platinum containing compound, lipid or protein kinase targeting agents, protein or lipid phosphatase targeting agents, anti-angiogenic agents, agents that induce cell differentiation, bradykinin 1 receptor and angiotensin II antagonists, cyclooxygenase inhibitors, heparanase inhibitors, lymphokines or cytokine inhibitors, bisphosphanates, rapamycin derivatives, anti-apoptotic pathway inhibitors, apoptotic pathway agonists, PPAR agonists, inhibitors of Ras isoforms, telomerase inhibitors, protease inhibitors, metalloproteinase inhibitors, aminopeptidase inhibitors.

For the treatment of oncologic diseases and solid tumors, compounds disclosed herein may be administered with an agent selected from the group comprising: dacarbazine (DTIC), alkylating agents (e.g., melphalan) anthracyclines (e.g. doxorubicin), corticosteroids (e.g. dexamethasone), Akt inhibitors (e.g. Perifosine), aromatase inhibitors, antiestrogen, anti-androgen, or gonadorelin agonists, anti-angiogenic agents (including VEGF inhibitors such as vandetanib, motesanib, axitinib, sorafenib, antibodies such as bevacizumab (Avastin), antibody derivatives such as ranibizumab (Lucentis), and the EGFR inhibitor gefitinib), topoisomerase 1 and 2 inhibitors, microtubule active agents, alkylating agents (e.g. cyclophosphamide, temozolomide), nucleoside or nucleotide analogues (e.g. 5-fluorouracil), antineoplastic antimetabolite, or platinum containing compounds, MITC, nitrosoureas, taxanes, lipid or protein kinase targeting agents, protein or lipid phosphatase targeting agents, , IMiDs (e.g. thalidomide, lenalidomide), protease inhibitors (e.g. bortezomib, NPI0052), IGF-1 inhibitors, CD40 antibody, Smac mimetics (e.g. telomestatin), FGF3 modulators (e.g. CHIR258), mTOR inhibitors (Rad 001), HDAC inhibitors (e.g. SAHA, Tubacin), IKK inhibitors, P38MAPK inhibitors, HSP90 inhibitors (e.g. 17-AAG), and other multikinase inhibitors (e.g. sorafenib).

In any case, the multiple therapeutic agents (at least one of which is a compound disclosed herein) may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may be any duration of time ranging from a few minutes to four weeks.

Thus, in another aspect, certain embodiments provide methods for treating disorders and symptoms relating to metal toxicity in a human or animal subject in need of such treatment comprising administering to said subject an amount of a compound disclosed herein effective to reduce or prevent said disorder in the subject, in combination with at least one additional agent for the treatment of said disorder that is known in the art. In a related aspect, certain embodiments provide therapeutic compositions comprising at least one compound disclosed herein in combination with one or more additional agents for the treatment of disorders and symptoms relating to metal toxicity.

The compounds, compositions, and methods disclosed herein are useful for the treatment of cancer. Specific cancers to be treated by the compounds, compositions, and methods disclosed herein include cancers of the breast, prostate, lung, colon, ovary, pancreas, liver, thyroid, stomach, uterine, lymphoma, brain (including, e.g., neuroblastoma and glioblastoma), skin, kidney, mouth, throat, tongue, and bladder, as well as leukemia. The cancer may be hormone-dependent or hormone-resistant, such as in the case of breast cancers. In certain embodiments, the cancer is a solid tumor. In certain embodiments, the cancer is and a drug resistant phenotype of a cancer disclosed herein or known in the art.

Besides being useful for human treatment, certain compounds and formulations disclosed herein may also be useful for veterinary treatment of companion animals, exotic animals and farm animals, including mammals, rodents, and the like. More preferred animals include horses, dogs, and cats.

### Estrogen Receptor Modulators

Methods of effecting estrogen receptor modulation so as to increase the relative effect of ERβ in comparison to ERα are several. Most directly, a full or partial agonist or an upregulator of ERβ can be used. Additionally, ERα can be selectively downregulated or degraded.

Methods of identifying ERβ modulators are also known in the art, and may be used to screen or confirm the ERβ activity of compounds. For example, a cell-based Gal4-beta-lactamase reporter gene assay (GERTA) in CHO cells for the ligand-induced activation of the human ERβ may be used, optionally in an ultra high throughput 3456-well nanoplate format. See, e.g., Peekhaus NT et al., "A beta-lactamase-dependent Gal4-estrogen receptor beta transactivation assay for the ultra-high throughput screening of estrogen receptor beta agonists in a 3456-well format," Assay Drug Dev Technol., 2003 Dec, 1(6):789-800. Alternatively or in conjunction, an in vitro assay may be used. In one such assay, upon agonist binding to a FLAG-tagged ERα or ERβ ligand binding domain (LBD), a biotinylated coactivator peptide is recruited to FLAG-tagged ER LBD to form a complex and thus allow fluorescence resonance energy transfer (FRET) to occur between europium in a europium-labeled anti-FLAG antibody and streptavidin-conjugated allophycocyanin. Compounds with estrogen antagonism block the agonist-mediated recruitment of a coactivator and prevent FRET, allowing for assessment of antagonist activity. See, e.g., Liu, J. et al., "A homogeneous in vitro functional assay for estrogen receptors: coactivator recruitment," Mol Endocrinol, 2003 Mar., 17(3):346-55. In certain embodiments, compounds will

Examples of ERβ agonists include 3β-adiol (5α-androstane-3β,17β-diol or 3β-androstanediol), DPN (2,3-bis(4-hydroxyphenyl)-propionitrile or diarylpropionitrile), apigenin (5,7-Dihydroxy-2-(4-hydroxyphenyl)-4*H*-1-benzopyran-4-one or 4',5,7-trihydroxyflavone), ERB-041 (7-Ethenyl-2-(3-fluoro-4-hydroxyphenyl)-5-benzoxazolol), FERB-033 (2-Chloro-3'-fluoro-3,4'-dihydroxy-[1,1-biphenyl]-4-carboxaldehyde oxime), liquirtigenin ((*S*)-2,3-Dihydro-7-hydroxy-2-(4-hydroxyphenyl)-4*H*-1-benzopyran-4-one), and WAY-00005 (7-Bromo-2-(4-hydroxyphenyl)-1,3-benzoxazol-5-ol). Additionally, antiestrogens such as tamoxifen and selective estrogen receptor down-regulators such as fulvestrant (Faslodex or ICI 182,780) may be used.

Yet further, because certain inhibitors of the cholesterol synthetic pathway have been found to selectively degrade the ERα receptor, agents which interact non-selectively with ERα and ERβ, such as estradiol, may be used and the anti-estrogenic effect of ERβ increased.

### Cholesterol Biosynthesis Inhibitors

The cholesterol biosynthesis inhibitor may be, for example, an inhibitor of oxidosqualene cyclase, squalene monooxygenase, squalene synthase, farnesyl pyrophosphate synthase, geranyl pyrophosphate synthase, mevalonate-5-pyrophosphate decarboxylase, phosphomevalonate kinase, or mevalonate kinase. In certain embodiments, the inhibitor of cholesterol biosynthesis is an inhibitor of oxidosqualene cyclase ("OSC"), for example, Ro 48-8071.

Cholesterol biosynthesis inhibitors may be identified by methods known in the art. For example, microsomal assays employing human, rat, or other liver microsomes may be used to identify OSC inhibitors, wherein the amount of radiolabeled 2,3-(S)-oxidosqualene consumed or lanosterol produced by microsomes treated with test compound measured and compared with control. See, e.g., Oliaro-Bosso S et al., J Enzyme Inhib Med Chem., 2009 Apr; 24(2):589-98, . WO199706802A1 (esp. pp. 19-21), WO199611201A1 (esp. pp. 335-336) Alternatively, whole cell assays may be used to measure cholesterol biosynthesis. For example, liver cells may be incubated with test compound and a radiolabeled cholesterol biosynthetic pathway substrate such as acetic acid or mevalonic acid, and the cholesterol produced (or perhaps the substrate consumed) may be measured according to methods known in the art and compared to control. See, e.g., WO199611201A1 (esp. pp. 336-341). Animal studies may also be used to measure, for example, liver or serum cholesterol in treated versus untreated subjects. See, e.g., WO199611201A1 (esp. pp. 335-341), WO199706802A1 (esp. pp. 19-21), and EP01346994.

Additional examples of OSC inhibitors are given below in Table 1.

**Table 1.**

| **Structure** | **OSC IC₅₀ (nM)** |
|---|---|
| 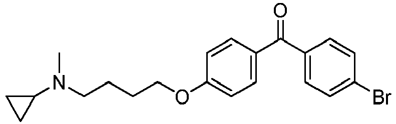 | 19 |
| 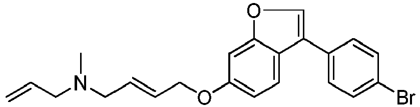 | 500 (83.3) |
| 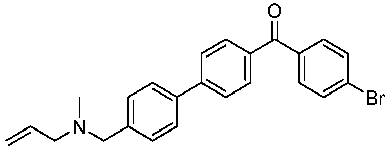 | 16 |
| 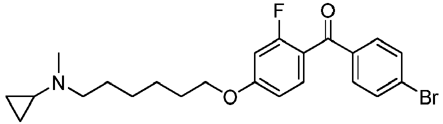 | 98 |
| 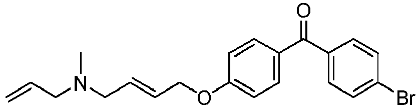 | 3.0 |
| 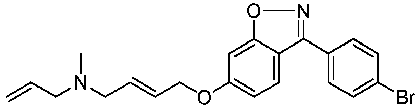 | 29 |
| 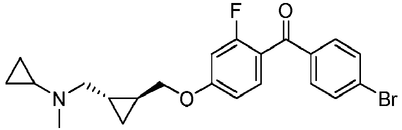 | 439 |
| 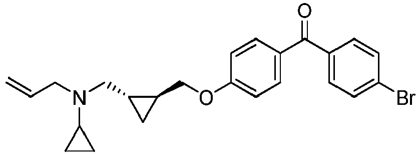 | 223 |
| 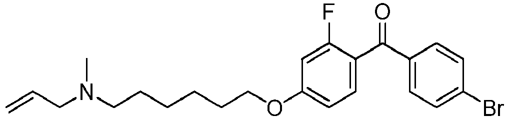 | 6.5 |
| 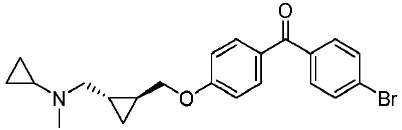 | 22 |
| 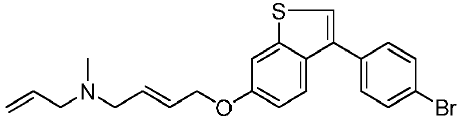 | 13.5 |
| 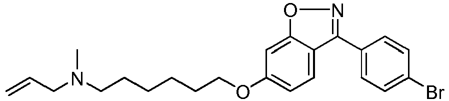 | 380 (79.0) |
| 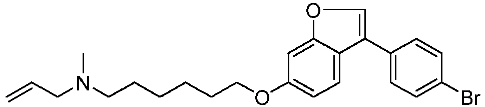 | 610 (86.0) |
| 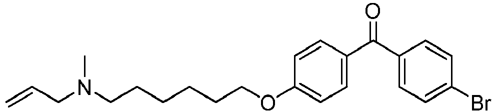 | 5.4 |
| 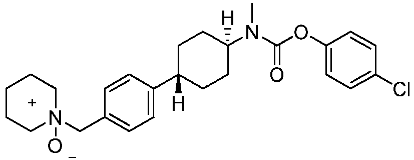 | 640 (86.5) |
| 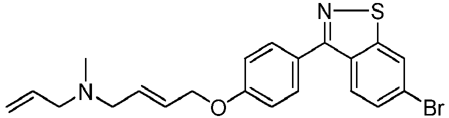 | 1860 (94.9) |
| 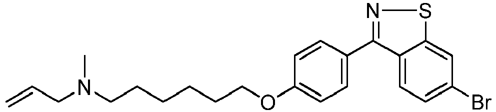 | 1900(95.0) |
| 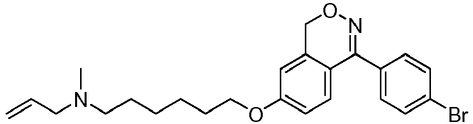 | 4.1 |
| 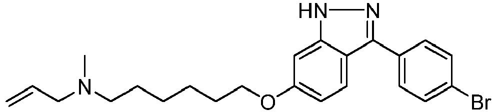 | 39 |
| 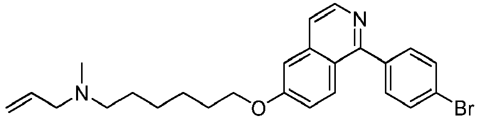 | 3.5 |
| 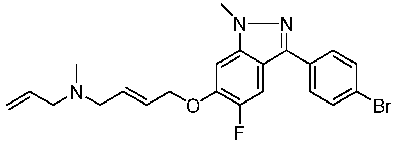 | 29 |
| 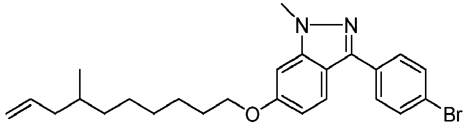 | 19.6 |
| 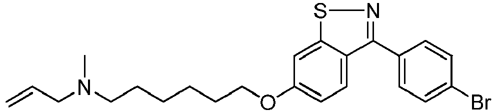 | 2.9 |
| 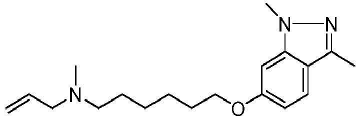 | 240 (70.4) |
| 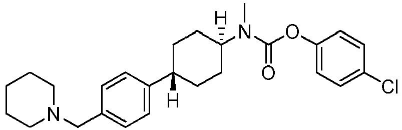 | 11.3 |
| 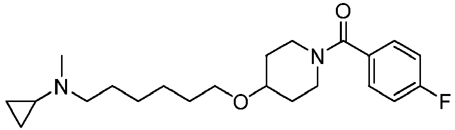 | 48 |
| 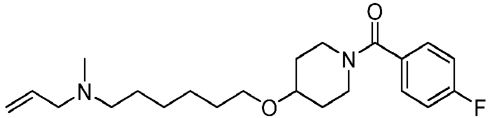 | 71 |
| 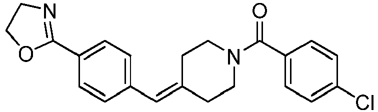 | 36 |

This table was adapted from Lenhart A et al., Binding Structures and Potencies of oxidosqualene cyclase inhibitors with the homologous squalene-hopene cyclase. J. Med. Chem. 2003, 46:2083-2092. One of the compounds above, Ro-48-8071, may be synthesized as described in [5], excerpts of which are provided below.

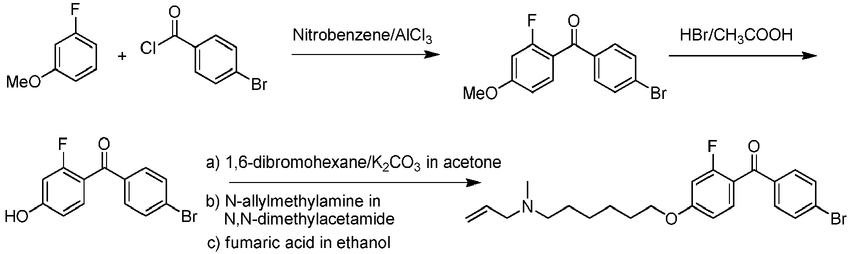

Ro 48-8071 (fumarate, MW = 564.45) was synthesized as shown above. All intermediates and Ro 48-8071 were characterized by 250 MHz ¹HNMR, IR, MS, and microanalyses. Melting points (uncorrected) were determined using a Biichi 510 apparatus. Proton NMR spectra were recorded on a Bruker AC250 spectrometer, and δ values are given in ppm relative to tetramethylsilane. IR spectra of KBr pellets were recorded using a Nicolet 7199-FT IR spectrometer. Mass spectra (MS) were obtained using the pneumatically assisted electrospray technique (Perkin-Elmer Sciex, type API-111). Results of elemental analyses were within 0.3% of theoretical values.

### (4-Bromophenyl)-(2'-fluoro-4'-methoxyphenyl)-methanone [1]

Aluminum chloride (144 g, 1.08 mol) was added to 450 ml pre-cooled nitrobenzene keeping the temperature < 8°C. Then, a suspension of 219.5 g (1 mol) 4-bromobenzoyl chloride in 200 ml nitrobenzene was added over 20 min, followed 10 min later by 108.5 ml (0.95 mol) 3-fluoroanisole. The reaction mixture was warmed to room temperature overnight, mixed into iced water (1.51), and extracted with 3 X 11 dichloromethane. The three organic phases were washed sequentially with 2 X 11 water, pooled, and dried (Na₂SO₄). Evaporation (85°C, 1 Torr) provided a mixture of (4-bromophenyl)-(2-fluoro-4-methoxy-phenyl)-methanone and (4-bromophenyl)-(4'-fluoro-2'-methoxyphenyl)-methanone which was immediately dissolved in 300 ml ethyl acetate, and crystallized at room temperature. The crystals were filtered off and washed with 100 ml ethyl acetate and 3 X 100 ml cyclohexane to give pure (4-bromophenyl)-(2'-fluoro-4'-methoxyphenyl)-methanone (122.4 g, 41.6%): mp 125-126°C; IR 1643 cm⁻¹; ¹HNMR (CDCl₃) δ 3.87 (s,OCH₃), 6.66 (dd, J = 12.1, 2.4 Hz 1H, 3'-H) 6.80 (dd, J = 8.7, 2.4 Hz, 1H, 5'-H), 7.54-7.68 (m, 5H, arom H); EIMS *m*/*z* 308 (M⁺, 1 Br). Calculated analysis for C₁₄H₁₆OBrFO₂: C, 54.40; H, 3.26; F, 6.15; Br, 25.85. Found: C, 54.57; H, 3.35; F, 6.21; Br, 26.07.

### (4-Bromophenyl)-(2'-fluoro-4'-hydroxyphenyl)-methanone [2]

A suspension of 61.8 g (200 mmol) of **[1]** in 400 ml acetic acid was treated with 230 ml 62%-aqueous hydrobromic acid, and stirred at 125°C for 8 h prior to evaporation. The residue was dissolved in 500 ml ethyl acetate and washed with 300 ml saturated sodium bicarbonate and 300 ml 10%-sodium chloride solution. The aqueous phases were extracted with 2 X 500 ml ethyl acetate. The organic phase was dried (Na₂SO₄,) and evaporated to give orange crystals of (4-bromophenyl)-(2'-fluoro-4'-hydroxyphenyl)-methanone [2] (57.2 g, 96.9%): mp 62-63°C; IR 1652 cm⁻¹; ¹HNMR (DMSO- 6.66 (dd, J = 12.1, 2.4 Hz 1H, 3'-H), 6.80 (dd, J = 8.7, dtj) a 6.68 (dd, J = 12.6, 2.2 Hz, 1H, 3'-H), 6.77 (dd, J = 8.5, 2.2 Hz, 1H, 5'-H), 7.49 (dd, J = 8.5, 8.5 Hz, 1H, 6'-H), 7.64 and 7.75 (AA'BB', 4H, 2,3,5,6-H), 10.85 (br s, 1H, OH); EIMS *m*/*z* 294 (M⁺, 1Br). Calculated analysis for C₁₃H₈BrFO₂: C, 52.91; H, 2.73; F, 6.44; Br, 27.08. Found: C, 52.94; H, 2.74; F, 6.42; Br, 26.84.

### [4'-(6-Allyl-methyl-amino-hexyloxy)-2'-fluorophenyl]-(4-bromophenyl)-methanone fumarate [3]

A mixture of 35.4 g (120 mmol) **[2]**, 54.9 ml (360 mmol) 1,6-dibromohexane and 49.8 g (360 mmol) potassium carbonate in 1100 ml acetone was vigorously stirred at 75°C for 5 h. After filtration and evaporation, the residue was dissolved in dichloromethane treated with sodium sulfate, filtered again, and evaporated. Crystallization with 400 ml cyclohexane-hexane 1:3 (v/v) first at 0°C and then at -78°C gave 53.2 g (116 mmol) crude [ 4'-(6-bromo-hexyloxy)-2'-fluorophenyl]-(4-bromophenyl)-methanone. This product was dissolved in 390 ml N,N-dimethylacetamide, cooled to 0°C, and 22.5 ml (232 mmol) N-allylmethylamine was added dropwise. After 22 h at room temperature the reaction was cooled to 0°C, and treated again with 22.5 ml (232 mmol) N-allylmethylamine. After 5 h the solution was evaporated (70°C, 1 Torr), neutralized with 300 ml saturated sodium bicarbonate, and extracted with 3 X 400 ml dichloromethane. The organic phase was dried (Na₂SO₄) evaporated to dryness, and purified by flash column chromatography (silica gel 0.04-0.063 mm, dichloromethane-methanol 95:5 (v/v), producing 37.7 g (84.1 mmol) of [4'-(6-allylmethyl-amino-hexyloxy)-2'-fluorophenyl]-(4-bromophenyl)-methanone. The free amine and 8.8 g (75.7 mmol) of fumaric acid were dissolved in 200 ml ethanol, evaporated, and crystallized from acetone-ethylacetate-ether to give [4'-(6-allylmethyl-amino-hexyloxy)-2'-fluoro-phenyl]-(4-bromophenyl)-methanone fumarate **[3]** (36.2 g,53.4%): mp 86-88°C; IR 1653 cm⁻¹; ¹HNMR (DMSO-_{δ6})δ 1.25-1.60 (m, 6H, OCH₂CH₂*CH₂CH₂CH₂*CH₂N), 1.70-1.80 (m, 2H, OCH₂*CH₂*CH₂CH₂CH₂CH₂N), 2.24 (s, 3H, NCH₃), 2.40-2.50 (m, 2H, OCH₂CH₂CH₂CH₂CH₂*CH₂*N), 3.11 (d, J = 6.5 Hz, 2H, N*CH₂*CHCH₂), 4.08 (t, J = 6.4 Hz, 2H, O*CH₂*CH₂CH₂CH₂CH₂CH₂N), 5.1 7-5.27 (m, 2H, NCH₂CH*CH₂*), 5.75-5.90 (m, 1H, NCH₂*CH*CH₂), 6.67 (s, 2H, fumarate), 6.91-7.00 (m, 2H, 3',5'-H), 7.56 (dd, J = 8.6, 8.6 Hz,1H,6'-H), 7.65 and7.76 (AA'BB', 4H, 2,3,5,6-H); EIMS *m*/*z* 448 (M⁺, 1Br). Calculated analysis for C₂₃H₂₇NBrFO₂·C₄H₄O₄; C, 57.45; H, 5.54; N, 2.48; F, 3.37; Br, 14.16. Found: C, 57.39; H, 5.57; N, 2.50; F, 3.38; Br, 14.15.

Additional examples of OSC inhibitors are given below. One such inhibitor is U18666A (also known as 3-beta-(2-(diethylamino)ethoxy)androst-5-en-17-one), shown below: 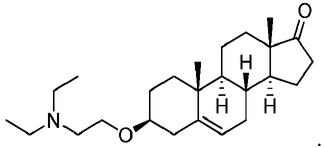 Although U18666A is a potent inhibitor of OSC, its therapeutic use may be limited by possible toxicological effects including cataract formation.

Yet additional examples of OSC inhibitors may be found in WO1996/011021A1, "Substituted heterobicyclic alkyl amines and their use as squalene oxide cyclase inhibitors" and EP1346994A1, "cholesterol biosynthesis inhibitors containing as the active ingredient tricyclic spiro compounds." Those compounds may be tested as disclosed herein for anticancer activity.

### Induction of ERβ and Reduction of ERα Levels in Breast Cancer Cells by a Cholesterol Biosynthesis Inhibitor: Western Blot Analysis

Cells used in these experiments included both the ERα+ve as well as ERα-ve cells, including triple negative types which are very aggressive growing. The ERα+ve cells used were T47-D and BT-474 cells. The ERα-ve cells used were MDA-MB-231, BT-20 and SkBr-3 cells. All cells were grown in media as specified in the data sheet from A TCC, provider of cell lines.

*Western blot analysis.* Whole cell extracts of breast cancer cells were prepared using a whole cell extract kit (Active Motif, Carlsbad, CA). Briefly, after treatment with Ro 48-8071, cells were washed once with PBS, harvested with 0.05% trypsin-EDT A, and centrifuged at 200g at 4°C for 5 min. Cell pellets were resuspended in lysis buffer and supernatant was transferred into pre-chilled microcentrifuge tubes and incubated on ice for 30 minutes. Samples were centrifuged at 14,000 x g at 4°C for 20 minutes, and supernatant was transferred to pre-chilled microcentrifuge tubes. Aliquots of samples were stored at -80°C.For western blot analysis, samples containing 50 µg of protein were separated in a NuPAGE 10% Bis-Tris Gel (Invitrogen, Carlsbad, CA).

Electrophoresis was performed at 120 V for 2 h using NuPAGE MES-SDS Running Buffer. Separated proteins were transferred to polyvinylidene difluoride membranes (BioRad Laboratories, Hercules, CA) at 35 V for 1.5 h.

Blots were blocked at room temperature (RT) for 1 hr in TBS containing 0.1% Tween 20 (TBS-T) and 5% nonfat dry milk and incubated with antibodies for ERα (dilution 1 :200) for 2 h at room temperature, and ERβ (dilution 1:150) at 4°C overnight. Blots were washed 3 times with TBS-T and incubated with secondary antibody for 1 h at RT before being washed a further 7 times with TBS-T.

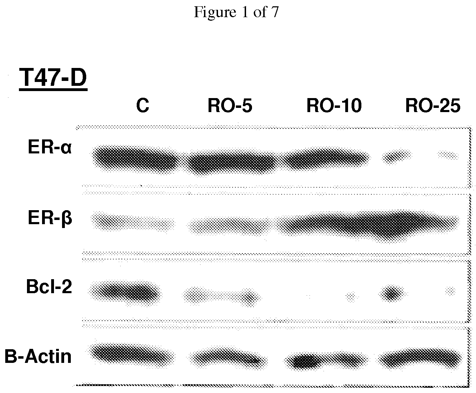

Immunoreactive bands were visualized using an ECL Plus detection kit (Amersham, Pharmacia Biotech, Arlington Heights, IL). Membranes were stripped and reblotted for beta-actin (Sigma), which was used as a control for protein loading. β-actin was used as a loading control to determine amount of total protein loaded in each lane. Results are shown in Figures 1-4, and demonstrate that Ro 48-8071 treatment induces the anti proliferative protein estrogen receptor-beta (ERβ) and reduces the pro-proliferative ERα protein in breast cancer cells.

Figure 1 shows the effect of Ro 48-8071 treatment for six hours on expression of ERα, ERβ, and Bcl-2 protein in T47-D breast cancer cells by Western blot. ERβ was induced, and ERα suppressed, by the cholesterol biosynthesis inhibitor Ro 48-8071 in a dose-dependent manner. Additionally, the survival protein Bcl-2 decreased with increasing dose of Ro 48-8071.

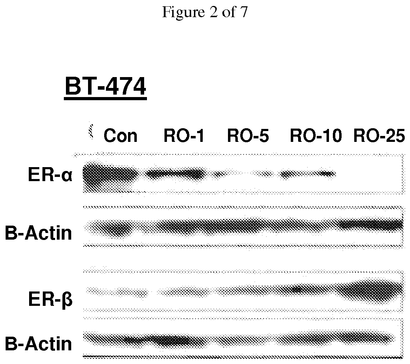

Figure 2 shows the effect of Ro 48-8071 treatment for six hours on expression of ERα and ERβ protein in BT-474 breast cancer cells. Similarly, ERβ was induced, and ERα suppressed, by the cholesterol biosynthesis inhibitor Ro 48-8071 in a dose-dependent manner.

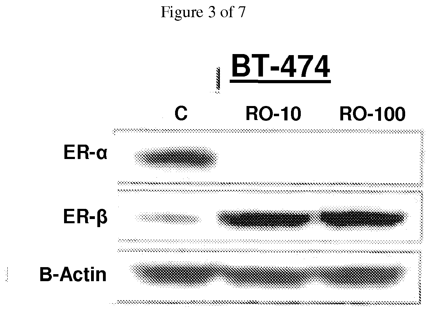

Figure 3 shows the effect of treatment with low dose Ro 48-8071 for seven days on expression of ERβ. ERβ was induced, and ERα suppressed, by the cholesterol biosynthesis inhibitor Ro 48-8071, to a similar degree when treated with either 10 or 100 nM Ro 48-8071. This indicates that a prolonged treatment course with a low dose of a cholesterol biosynthesis inhibitor may be effective in treating cancer.

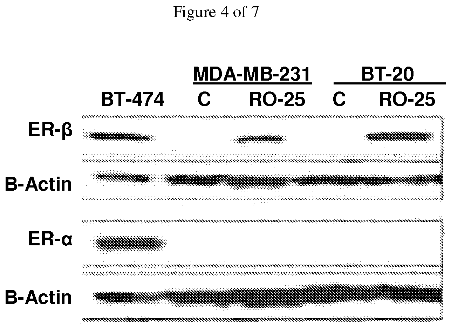

Figure 4 shows that Ro 48-8071 stimulates expression of ERβ in ERα-negative (triple-negative) BT-20 and MDA-MB-231 breast cancer cell lines. This indicates that treatment with a cholesterol biosynthesis inhibitor may open a new avenue of treatment in triple-negative breast cancer, by making cells susceptible to treatment with ERβ modulators.

### ERβ siRNA Interference Abrogates the Inducing Effects of Ro and Reduces ERβ levels

ERβ siRNA (human) and control siRNA were obtained from Santa Cruz Biotechnology, Inc. (Santa Cruz, CA, e.g. catalog no.s SC-35325 and SC-37007). ERβ siRNA is a pool of 3 target-specific 20-25 nt siRNA designed to knock down ERβ gene expression.

Transfection reagent, oligonucleotides and medium, Opti-MEM were from Invitrogen (Carlsbad, CA). The day before transfection, cells were seeded in six-well plates at a density of 8 x 10^4 cells per well with 10% FBS DMEM/F12 medium.

To make solution A, ERβ siRNA was diluted in Opti-MEM, resulting in a final concentration of 30 or 60 nM in 100µl/well and incubate for 5 min. at room temperature (RT). To make solution B, transfection regent RNAiMAX (5µL/well) was diluted in Opti-MEM to 100µl/well and incubated for 5 min at RT. Solution A (diluted ERβ siRNA) was added directly to solution B (diluted transfection reagent RNAiMAX) using a pipette and mixed gently by pipetting the solution up and down. The mixture was incubated for 20-30 minutes at RT. For each transfection, 0.8 ml 10% FBS DMEM/F12 medium was added to each tube containing the siRNA and transfection reagent mixture (Solution A+ Solution B). This was mixed gently and 1 ml was added to each well containing cells, which was then washed with 2 ml/well serum free DMEM/F12 medium. Cells were incubated for 24 hrs, after which 1 ml fresh 10% FSB DM EM/F12 medium was added to each well. Cells were then incubated for a further 24 to 48 hrs. Cells were then treated with Ro 48-8071 at different concentrations and a time course study performed for Western Blot analysis or cell growth assay (SRB assay).

Western blot analysis (procedure above) demonstrated lower levels of ERβ following siRNA treatment. Additionally, use of an SRB assay enabled monitoring of the effects of loss of ERβ on mediation of cell viability by Ro 48-8071. In sum, reduction of ERβ levels using siRNA led to an abrogation of the inducing effects of Ro 48-8071, further supporting the notion that. Results are shown in Figure 5.

### Combination Therapy with ERβ Agonist and OSC Inhibitor Causes Additive Loss of Tumor Cell Viability

- **ERβ agonist potentiates the loss of tumor cell viability induced by Ro 48-8071 as determined by sulforhodamine B (SRB) cell viability assay.**
- **ERβ antagonist prevents the loss of tumor cell viability induced by Ro 48-8071.**

The sulforhodamine B (SRB) assay was employed to evaluate the effect potent cholesterol biosynthesis inhibitor Ro 48-8071 alone or in combination with ERβ-specific ligands on viability of breast cancer cells, with slight modification of the original procedure established at NCI. Ro 48-8071, and ERβ-specific ligands (either an agonist DPN (diarylpropionitrile), or an antagonist PHTPP (4-[2-phenyl-5,7-bis(trifluoromethyl) pyrazolo[1,5-a]pyrimidin-3-yl]phenol)) ("PH") were used in BT-474, T47-D, MDA-MB-231, and BT-20 human breast cancer cells in this study.

Accordingly, cells were seeded in 96-well plates in 100 µL culture medium and incubated overnight at 37°C with 5% CO₂. Culture medium was removed after 24 h, and the attached cells were washed with DMEM/F12 medium and treated by Ro 48-8071 alone or with ERβ-specific ligands at various concentrations for 24-48 hrs. treated with 10 µM Ro 48-8071 ± 1 µM DPN, or with DPN alone

The surviving or adherent cells were fixed in situ by withdrawing the growth medium and adding 100 µL of PBS and 100 µL of 50% cold trichloroacetic acid and then incubating at 4°C for 1 h. Attached cells (that had survived) were washed 5 times with ice-water, dried at room temperature (RT), and then stained with 50 µL 4% SRB for 8 min at room temperature (RT). Unbound dye was removed by washing 5 times with cold 1% acetic acid, and the plates were dried at RT. Bound stain was solubilized with 150 µL 10 mM Tris buffer, and absorbance of samples measured at 520 nm with a SpecTRA MAX 190 microplate reader (Sunnyvale, CA). Six wells were used for each concentration, and each experiment was performed at least twice.

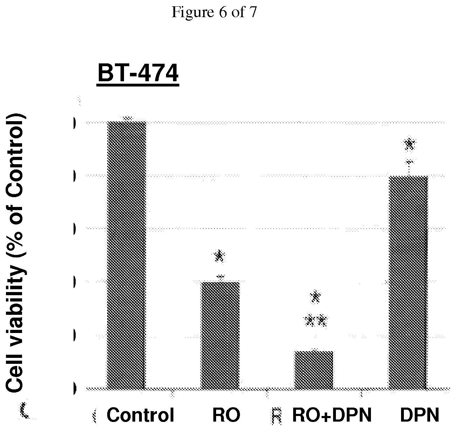

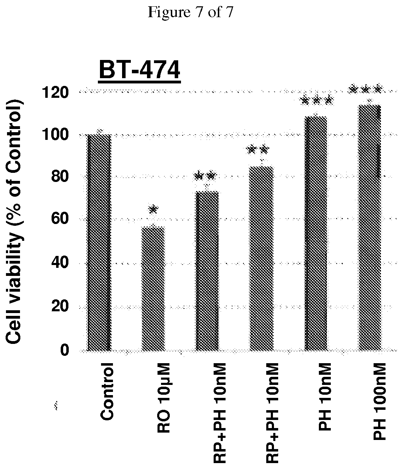

Combination therapy with Ro 48-8071 and ERβ agonist DPN increased cell death more effectively (additively). Conversely, the anti-proliferative effects of Ro 48-8071 in breast cancer cells is reduced when it is combined with ERβ antagonist PHTPP. See Figures 6 and 7, respectively. This demonstrates that cholesterol biosynthesis inhibitors could be used in combination with ERβ agonists to suppress breast tumor growth. This would be especially useful for triple negative tumor types where there are only limited treatment options.

### Additional Combinations

In addition to the studies and uses disclosed above, the combination of an inhibitor of cholesterol biosynthesis and an ERβ agonist may be tested according to the protocols described herein and as described in the art.

Ro 48-8071 may be used in combination with the ER-β agonists DPN, Liquiritigenin, apigenin and luteolin in ER+, PR+ breast cancer cell lines such as BT-474, MCF-7, and T47-D cells. It is expected that these combination treatments are additive or synergistic with respect to inhibition of cancer cell growth. Effects of the same combination of agents will also be examined in triple negative (ER-, PR-, and HER/2- negative) MDA-MB-231 and BT-20 breast cancer cells. Similarly, it is expected that these combination treatments will be additive or synergistic with respect to inhibition of cancer cell growth, demonstrating that inhibitors of cholesterol biosynthesis can induce ERβ and sensitize cancer cells, including EPβ- and triple-negative cells, to treatment.

Inhibitors of cholesterol biosynthesis such as Ro 48-8071 may also be combined with agents such as the clinically approved anti-hormones tamoxifen and fulvestrant (ICI 182,780; Faslodex), which exert anti-estrogenic effects and interact with both estrogen receptors (ERα and ERβ). The inhibitory effects of the aforementioned combinations may be examined in all the cell lines described above, including triple negative cells in which R0 48-8071 induces ERβ. As above, it is expected that these combination treatments will be additive or synergistic with respect to inhibition of cancer cell growth, demonstrating that inhibitors of cholesterol biosynthesis can induce ERβ and sensitize cancer cells, including ERβ- and triple-negative cells, to treatment.

The effects of combining inhibitors of cholesterol biosynthesis such as Ro 48-8071 with additional chemotherapeutic drugs, such as the commonly used docetaxel and doxorubicin, may also be examined in all the cell lines described above. These studies are expected to demonstrate that inhibitors of cholesterol biosynthesis sensitizes cells to chemotherapeutic drugs and/or increases the effectiveness of such drugs. It is expected that this could pave the way to using reduced doses of chemotherapeutic drugs, thereby reducing drug induced toxicity in cancer patients.

### In Vivo Studies

Xenograft models may be employed to further demonstrate the utility of the methods and combination therapies disclosed herein. In each of the following protocols, it is expected that significant differences in tumor volume will be seen between test and control groups, indicating that inhibitors of cholesterol biosynthesis and ERβ agonists used together are additively or synergistically effective at reducing tumor volume.

Animal weight may be monitored throughout these experiments. No significant difference was is expected between control and treated groups, which would indicate that the combination of inhibitors of cholesterol biosynthesis and ERβ agonists is non-toxic in tumor-bearing nude mice at doses used for inhibiting tumor growth. Varying concentrations of chemotherapeutics may be tested along with inhibitors of cholesterol biosynthesis and ERβ agonists in order to identify an acceptably non-toxic therapeutic dose.

### Breast Cancer

The exemplary inhibitor, Ro 48-8071, has also been evaluated in vivo and found to inhibit growing of human breast cancer xenografts in nude mice without toxicity. Similar protocols may be employed to test the efficacy of inhibitors of cholesterol biosynthesis and ERβ agonists used together.

Female athymic nu/nu nude mice, 5 to 6 weeks-old (18-22g) may be purchased from a number of suppliers, such as Harland Sprague-Dawley, Inc. Nude mice are inoculated with 17-β-estradiol pellets (1.7 mg/pellet, 60 days release) 48 hours before inoculating tumor cells. BT-474 human breast cancer cells, 5x10⁶ in 0.15 ml solution mixed with matrigel and DMEM/F12 medium (4/1, v/v) are injected into each flank of mouse subcutaneously and both flanks of each mouse are injected. When tumor volume reaches around 100 mm3, animal are randomly assigned to three groups and the treatment started with RO 48-8071 (5mg/kg/day or 10mg/kg/day) via tail-vein injection for five days, followed by same treatment every other day for five additional times. Animals in control group receive the vehicle alone under identical conditions. Tumor volumes are measured by a digital caliper and calculated using the formula (L x W x H) x 0.5236.

This protocol may be varied to investigate the effect of the combination of an inhibitor of cholesterol biosynthesis and an ERβ agonist on cancers arising from T47-D, MCF-7, HCC-1428, and ZR-75 breast cancer xenografts, and MDA-231 and BT-20 triple-negative breast cancer xenografts as well.

### Prostate Cancer

Male athymic nu/nu nude mice, 6 weeks-old (21-25g) may be purchased from Harlan Laboratories, Inc. Human prostate cancer PC-3 cells, 5x106 in 0.15 ml solution mixed with matrigel and DMEM/F12 medium (1/1, v/v) are injected into each flank of mouse subcutaneously, and both flanks of each mouse are injected. When tumor volume reaches around 100 mm3, animals are randomly assigned to three groups and the treatment started with Ro 48-8071 (5 mg/kg/day or 20 mg/kg/day) via tail-vein injection for five days, followed by same treatment every other day for seven additional times. Animals in control group receive the vehicle alone under identical conditions. Tumor volumes are measured every three days by a digital caliper and calculated using the formula (L x W x H) x 0.5236.

This protocol may be varied to investigate the effect of the combination of an inhibitor of cholesterol biosynthesis and an ERβ agonist on cancers arising from LNCaP or DU145 prostate cancer xenografts as well.

### Other Cancers

Other cancers may be investigated using variations on the xenograft models above. For example, nude mice may be inoculated with cells from: ovarian cancer cell lines OVCAR-3 or SK-OV-3;colon cancer cell lines DLD-1 or LoVo; lung cancer cell lines H69AR, NCI-H23, or A549; and pancreatic cancer cell lines Capan-1 or BxPC-3.

## Claims

1. An amount of cholesterol biosynthesis inhibitor sufficient to induce ERβ in cancer cells and
(i) a therapeutically effective amount of a selective or nonselective ERβ agonist and (ii) a therapeutically effective amount of an antihormone for use in the treatment of cancer in a subject,
wherein the cholesterol biosynthesis inhibitor and selective or nonselective ERβ agonist and antihormone are administered sequentially or concurrently.

2. An amount of cholesterol biosynthesis inhibitor sufficient to induce ERβ in cancer cells and a subtherapeutic amount of a chemotherapeutic drug for use in the treatment of a disease responsive to chemotherapy,
wherein the cholesterol biosynthesis inhibitor and subtherapeutic amount of a chemotherapeutic drug are administered sequentially or concurrently.

3. A pharmaceutical composition comprising a cholesterol inhibitor and a selective or nonselective ERβ agonist, and optionally an antihormone.

4. A kit comprising:
i) a cholesterol biosynthesis inhibitor formulated for administration to a patient;
ii) a therapeutically effective amount of a selective or nonselective ERβ agonist formulated for administration to a patient, and optionally additionally a therapeutically effective amount of an antihormone formulated for administration to a patient; and
iii) instructions for administration to a patient.

5. The composition as recited in claim 3, wherein the cholesterol inhibitor is an inhibitor of cholesterol biosynthesis.

6. The combination for use, composition or kit as recited in any one claims 1, 2, 4 or 5, wherein the cholesterol biosynthesis inhibitor is an inhibitor of OSC.

7. The combination for use, composition or kit as recited in claim 6, wherein the inhibitor of OSC is Ro 48-8071.

8. The cholesterol biosynthesis inhibitor or chemotherapeutic drug for use as recited in claim 2, wherein the disease is cancer.

9. The combination for use of claim 1 or claim 8, wherein the cancer is breast cancer, ovarian cancer, prostate cancer or lung cancer.

10. The combination for use of claim 9, wherein the cancer is ERβ-negative breast cancer or triple negative breast cancer.

11. The combination for use, composition or kit as recited in any of claims 1, 3 or 4, wherein the ERβ agonist is a selective ERβ agonist.

12. The combination for use, composition or kit as recited in any of claims 1, 3 or 4, wherein the ERβ agonist is chosen from 3β-adiol, DPN, apigenin, ERB-041, FERB-033, liquirtigenin, and WAY-00005.

13. The combination for use, composition or kit as recited in any of claims 1, 3 or 4, wherein the antihormone is an antiestrogen.

14. The combination for use, composition or kit as recited in claim 13, wherein the antiestrogen is chosen from tamoxifen and fulvestrant.

15. The cholesterol biosynthesis inhibitor or chemotherapeutic drug for use as recited in claim 2, wherein the chemotherapeutic drug is chosen from doxorubicin and docetaxel.

16. The kit as recited in claim 4, wherein both the ERβ agonist and the antihormone are administered.

## Patentansprüche

1. Cholesterinbiosynthesehemmer in einer für eine Aktivierung von ERβ in Krebszellen ausreichenden Menge und
(i) eine therapeutisch wirksame Menge eines selektiven oder nichtselektiven ERβ-Agonisten und (ii) eine therapeutisch wirksame Menge eines Antihormons zur Verwendung bei der Behandlung von Krebs in einem Individuum,
wobei der Cholesterinbiosynthesehemmer und der selektive oder nichtselektive ERβ-Agonist und das Antihormon nacheinander oder gleichzeitig verabreicht werden.

2. Cholesterinbiosynthesehemmer in einer für eine Aktivierung von ERβ in Krebszellen ausreichenden Menge und eine subtherapeutische Menge eines chemotherapeutischen Arzneimittels zur Verwendung bei der Behandlung einer auf Chemotherapie ansprechenden Erkrankung,
wobei der Cholesterinbiosynthesehemmer und die subtherapeutische Menge eines chemotherapeutischen Arzneimittels nacheinander oder gleichzeitig verabreicht werden.

3. Pharmazeutische Zusammensetzung, umfassend einen Cholesterinhemmer und einen selektiven oder nichtselektiven ERβ-Agonisten sowie wahlweise ein Antihormon.

4. Kit, umfassend:
i) einen für eine Verabreichung an einen Patienten formulierten Cholesterinbiosynthesehemmer,
ii) eine für eine Verabreichung an einen Patienten formulierte therapeutisch wirksame Menge eines selektiven oder nichtselektiven ERβ-Agonisten sowie wahlweise zusätzlich eine für eine Verabreichung an einen Patienten formulierte therapeutisch wirksame Menge eines Antihormons und
iii) Anweisungen für eine Verabreichung an einen Patienten.

5. Zusammensetzung nach Anspruch 3, wobei es sich bei dem Cholesterinhemmer um einen Hemmer der Cholesterinbiosynthese handelt.

6. Kombination zur Verwendung, Zusammensetzung oder Kit nach einem der Ansprüche 1, 2, 4 oder 5, wobei es sich bei dem Cholesterinbiosynthesehemmer um einen OSC-Hemmer handelt.

7. Kombination zur Verwendung, Zusammensetzung oder Kit nach Anspruch 6, wobei es sich bei dem OSC-Hemmer um Ro 48-8071 handelt.

8. Cholesterinbiosynthesehemmer oder chemotherapeutisches Arzneimittel zur Verwendung nach Anspruch 2, wobei es sich bei der Erkrankung um Krebs handelt.

9. Kombination zur Verwendung nach Anspruch 1 oder Anspruch 8, wobei es sich bei dem Krebs um Brustkrebs, Eierstockkrebs, Prostatakrebs oder Lungenkrebs handelt.

10. Kombination zur Verwendung nach Anspruch 9, wobei es sich bei dem Krebs um ERβ-negativen Brustkrebs oder dreifach negativen Brustkrebs handelt.

11. Kombination zur Verwendung, Zusammensetzung oder Kit nach einem der Ansprüche 1, 3 oder 4, wobei es sich bei dem ERβ-Agonisten um einen selektiven ERβ-Agonisten handelt.

12. Kombination zur Verwendung, Zusammensetzung oder Kit nach einem der Ansprüche 1, 3 oder 4, wobei der ERβ-Agonist gewählt ist aus 3β-Adiol, DPN, Apigenin, ERB-041, FERB-033, Liquiritigenin und WAY-00005.

13. Kombination zur Verwendung, Zusammensetzung oder Kit nach einem der Ansprüche 1, 3 oder 4, wobei es sich bei dem Antihormon um ein Antiöstrogen handelt.

14. Kombination zur Verwendung, Zusammensetzung oder Kit nach Anspruch 13, wobei das Antiöstrogen ausgewählt ist aus Tamoxifen und Fulvestrant.

15. Cholesterinbiosynthesehemmer oder chemotherapeutisches Arzneimittel zur Verwendung nach Anspruch 2, wobei das chemotherapeutische Arzneimittel ausgewählt ist aus Doxorubicin und Docetaxel.

16. Kit nach Anspruch 4, wobei sowohl der ERβ-Agonist als auch das Antihormon verabreicht werden.

## Revendications

1. Quantité d'inhibiteur de la biosynthèse du cholestérol suffisante pour induire le récepteur bêta aux oestrogènes (ERβ) dans des cellules cancéreuses et
(i) une quantité thérapeutiquement efficace d'un agoniste d'ERβ sélectif ou non sélectif et
(ii) une quantité thérapeutiquement efficace d'une antihormone destinée à être utilisée dans le traitement du cancer chez un sujet,
dans laquelle l'inhibiteur de la biosynthèse du cholestérol, l'agoniste d'ERβ sélectif ou non sélectif et l'antihormone sont administrés de manière séquentielle ou simultanée.

2. Quantité d'inhibiteur de la biosynthèse du cholestérol suffisante pour induire ERβ dans des cellules cancéreuses et une quantité sous-thérapeutique d'un médicament chimiothérapeutique destiné à être utilisé dans le traitement d'une maladie qui répond à la chimiothérapie, dans laquelle l'inhibiteur de la biosynthèse du cholestérol et la quantité sous-thérapeutique d'un médicament chimiothérapeutique sont administrés de manière séquentielle ou simultanée.

3. Composition pharmaceutique comprenant un inhibiteur du cholestérol et un agoniste d'ERβ sélectif ou non sélectif, et facultativement une antihormone.

4. Trousse comprenant :
i) un inhibiteur de la biosynthèse du cholestérol préparé pour l'administration à un patient ;
ii) une quantité thérapeutiquement efficace d'un agoniste d'ERβ sélectif ou non sélectif préparé pour l'administration à un patient, et facultativement en plus une quantité thérapeutiquement efficace d'une antihormone préparée pour l'administration à un patient ; et
iii) des instructions pour l'administration à un patient.

5. Composition selon la revendication 3, dans laquelle l'inhibiteur du cholestérol est un inhibiteur de la biosynthèse du cholestérol.

6. Combinaison destinée à être utilisée, composition ou trousse selon l'une quelconque des revendications 1, 2, 4 ou 5, dans laquelle l'inhibiteur de la biosynthèse du cholestérol est un inhibiteur de l'OSC.

7. Combinaison destinée à être utilisée, composition ou trousse selon la revendication 6, dans laquelle l'inhibiteur de l'OSC est le Ro 48-8071.

8. Inhibiteur de la biosynthèse du cholestérol ou médicament chimiothérapeutique destiné à être utilisé selon la revendication 2, dans lequel la maladie est le cancer.

9. Combinaison destinée à être utilisée selon la revendication 1 ou la revendication 8, dans laquelle le cancer est le cancer du sein, le cancer de l'ovaire, le cancer de la prostate ou le cancer du poumon.

10. Combinaison destinée à être utilisée selon la revendication 9, dans laquelle le cancer est le cancer du sein à ERβ négatif ou le cancer du sein triple négatif.

11. Combinaison destinée à être utilisée, composition ou trousse selon l'une quelconque des revendications 1, 3 ou 4, dans laquelle l'agoniste d'ERβ est un agoniste d'ERβ sélectif.

12. Combinaison destinée à être utilisée, composition ou trousse selon l'une quelconque des revendications 1, 3 ou 4, dans laquelle l'agoniste d'ERβ est choisi parmi le 3β-adiol, le DPN, l'apigénine, l'ERB-041, le FERB-033, la liquirtigénine, et le WAY-00005.

13. Combinaison destinée à être utilisée, composition ou trousse selon l'une quelconque des revendications 1, 3 ou 4, dans laquelle l'antihormone est un anti-oestrogène.

14. Combinaison destinée à être utilisée, composition ou trousse selon la revendication 13, dans laquelle l'anti-oestrogène est choisi parmi le tamoxifène et le fulvestrant.

15. Inhibiteur de la biosynthèse du cholestérol ou médicament chimiothérapeutique destiné à être utilisé selon la revendication 2, dans lequel le médicament chimiothérapeutique est choisi parmi la doxorubicine et le docétaxel.

16. Trousse selon la revendication 4, dans laquelle l'agoniste d'ERβ et l'antihormone sont tous les deux administrés.
